# EUROPÄISCHE PATENTANMELDUNG

(11) **EP 3 753 519 A1**
(43) Veröffentlichungstag der Anmeldung: **23.12.2020**
(21) Anmeldenummer: 19181322.9
(22) Anmeldetag: 19.06.2019
(51) Int. Cl.: A61B 34/30, A61B 34/35, A61B 90/25, A61B 90/35, A61B 90/50, B25J 9/16, B25J 13/04, B25J 13/02, B25J 13/06, A61B 90/30, A61B 34/00, A61B 90/00

(54) **MEDIZINISCHE HANDHABUNGSVORRICHTUNG ZUR STEUERUNG EINER HANDHABUNGSVORRICHTUNG**

(71) Anmelder: Karl Storz SE & Co. KG, 78532 Tuttlingen (DE)
(72) Erfinder: Schrader, Stephan, 78532 Tuttlingen (DE); Köhler, Benedikt, 78532 Tuttlingen (DE); Chang-Hae KIM, 78532 Tuttlingen (DE); Schulze, Marco, 78532 Tuttlingen (DE)
(74) Vertreter: Witte, Weller & Partner Patentanwälte mbB

(57) **Zusammenfassung**

Die Offenbarung betrifft eine medizinische Handhabungsvorrichtung mit einem
einen Instrumentenhalter (40) zur Aufnahme eines Beobachtungsinstruments (38) mit einem Bildaufnehmer (118) zur Erfassung eines Bildausschnitts (116); einer
robotischen Handhabungseinheit (34), die den Instrumentenhalter (40) trägt; einer Steuereinrichtung (44) mit einer Handhabungssteuereinheit (46) zur Steuerung der robotischen Handhabungseinheit (34) und einer Instrumentensteuereinheit (48) zur Steuerung des Beobachtungsinstruments (38); und mit einem mit der Steuereinrichtung (44) gekoppelten Eingabegerät (50) zur Wahl eines wiederzugebenden Bildausschnitts (116), wobei die Steuereinrichtung (44) dazu ausgebildet ist, die robotische Handhabungseinheit (34) in Reaktion auf Bedienereingaben am Eingabegerät (50) zu steuern, um den erfassten Bildausschnitt (116) zu ändern, und wobei die Steuereinrichtung (44) dazu ausgebildet ist, Bedienbefehle am Eingabegerät (50) in Abhängigkeit von einer gegebenen Orientierung des Bildaufnehmers (118) in Bewegungsvorgaben zu überführen, insbesondere in Bewegungsvorgaben für die robotische Handhabungseinheit (34). Die Offenbarung betrifft ferner ein Verfahren zur Steuerung einer Handhabungsvorrichtung (10).

## Beschreibung

Die vorliegende Offenbarung betrifft eine medizinische Handhabungsvorrichtung und ein Verfahren zur Steuerung einer Handhabungsvorrichtung, wobei die Handhabungsvorrichtung eine robotische Handhabungseinheit aufweist, die einen Instrumentenhalter zur Aufnahme eines Instruments trägt, beispielsweise eines Beobachtungsinstruments, und wobei eine Handhabungssteuereinrichtung zur Steuerung der robotischen Handhabungseinheit vorgesehen ist, die über ein Eingabegerät steuerbar ist.

Aus der DE 10 2013 110 543 A1 ist ein als Beobachtungsinstrument gestaltetes medizinisches Instrument bekannt, das zum Aufnehmen eines Bildes eines Objektfelds an einem menschlichen oder tierischen Körper von außerhalb des Körpers ausgebildet ist, wobei das Instrument einen Schaft und eine an einem distalen Ende des Schafts angeordnete Beobachtungsoptik zum Aufnehmen des Bildes des Objektfelds aufweist, wobei die Beobachtungsoptik als Stereooptik mit mindestens einem elektronischen Bildaufnehmer zum Aufnehmen eines Stereobilds des Objektfelds ausgebildet ist, und wobei das Instrument eine Optikeinheit aufweist, die die Beobachtungsoptik umfasst, und die um eine zu einer Blickrichtung der Beobachtungsoptik näherungsweise parallele erste Drehachse drehbar ist.

Ein solches Beobachtungsinstrument zur Beobachtung eines Objektfeldes von außerhalb des Körpers wird als Exoskop bezeichnet. Ferner sind Instrumente, insbesondere Beobachtungsinstrumente, bekannt, die als Endoskope zum Aufnehmen eines Bildes innerhalb des menschlichen oder tierischen Körpers gestaltet sind.

Aus der DE 10 2015 121 017 A1 ist eine Beobachtungsvorrichtung bekannt, die ein Beobachtungsinstrument und ein als Mehrachs-Eingabemodul gestaltetes Eingabegerät zur Steuerung des Beobachtungsinstruments aufweist. Das Eingabegerät ist ähnlich einer sogenannten Space-Mouse gestaltet. Die DE 10 2015 121 017 A1 lehrt, das Eingabegerät sowohl zur Steuerung von Bildaufnahmeparametern als auch zur Steuerung von Bildwiedergabeparametern zu verwenden. Ein Bildaufnahmeparameter betrifft etwa eine Fokusverstellung. Ein Bildwiedergabeparameter betrifft etwa einen digitalen Zoom.

Im Sinne der vorliegenden Offenbarung ist ein distales Ende eines Elements ein einem Beobachtungsobjekt, etwa einem Patienten, zugewandtes Ende. Demgegenüber ist ein proximales Ende des Elements ein dem distalen Ende und folglich auch dem Beobachtungsobjekt abgewandtes Element. Bei einem handgeführten Instrument ist das proximale Ende regelmäßig dem Bediener des Instruments zugewandt. Bei einem mittels einer Handhabungseinheit geführten Instrument ist das Instrument zuweilen - aber nicht zwangsläufig - im Bereich seines proximalen Endes an der Handhabungseinheit aufgenommen, etwa an einem Gehäuse.

Ferner sind sogenannte Teleoperationssysteme oder Telemanipulationssysteme bekannt, etwa aus der US 5,696,837 A, bei denen ein Instrument in Form eines Beobachtungsinstruments oder dergleichen über einen Manipulator gehalten und ferngesteuert wird.

Medizinische Instrumente, etwa Beobachtungsinstrumente in Form eines Endoskops oder eines Exoskopes, können grundsätzlich handgehalten bzw. handgeführt sein. Dies hat den Vorteil, dass der Benutzer intuitiv und unmittelbar die Blickrichtung, das Objektfeld bzw. den Bildausschnitt und weitere Parameter der Bilderfassung anpassen kann, indem das Instrument entsprechend im Raum positioniert wird.

Es sind jedoch auch Systeme bekannt, bei denen Instrumente nicht handgehalten oder handgeführt werden, sondern an einem Stativ oder einem Ausleger aufgenommen sind. Dies hat den Vorteil, dass kein Bediener erforderlich ist, der das Instrument händisch in der gewünschten Position und Orientierung halten muss. Hierbei ist es vorstellbar, dass das Instrument ortsunveränderlich angeordnet ist, um beispielsweise während einer Operation permanent ein- und denselben Bildausschnitt in einem vorgewählten Objektfeld zu beobachten.

Darüber hinaus ist es auch vorstellbar, das Instrument an einer Handhabungseinheit bzw. einem Manipulator (auch als motorisches Haltesystem bzw. Roboter bezeichnet) anzuordnen, um Bewegungsfreiheitsgrade der Handhabungseinheit zur Bewegung und Ausrichtung des Instruments zu nutzen.

Auf diese Weise kann auch bei einem nicht unmittelbar handgehaltenen oder handgeführten Instrument eine Änderung der Position, Orientierung und/oder des Bildausschnitts erfolgen. Hierfür ist jedoch eine Bedienung erforderlich, um die gewünschte Bewegung des Instruments zu veranlassen.

Häufig sind jedoch bereits Bedienelemente für die Instrumente als solches vorgesehen, etwa Bedienelemente zur Steuerung von Bildaufnahmeparametern und/oder Bildwiedergabeparametern eines Bilderfassungssystems, welches ein Beobachtungsinstrument mit Bildaufnehmer und eine entsprechende Anzeige zur Bildwiedergabe aufweist. Das heißt, selbst ohne zusätzliche Bewegung des Instruments sind bereits diverse Bedienoperationen vorstellbar, für welche Bedienelemente vorgesehen sind.

Bei der Automatisierung bzw. maschinellen Unterstützung medizinischer Tätigkeiten ist jedoch darauf zu achten, dass die Systeme als solches noch intuitiv, einfach und sicher bedienbar sind. Insbesondere bei telemedizinischen Systemen bzw. robotischen Systemen muss bedacht werden, dass häufig keine unmittelbare Rückkopplung an den Bediener möglich ist. Dies führt etwa im Vergleich zur rein manuellen handgeführten Betätigung unter Umständen zu Fehlbedienungen, wenn dem Bediener nicht unmittelbar klar ist, welche Aktivität mit dem aktuell vorgenommenen Bedienbefehl ausgelöst wird.

Vor diesem Hintergrund liegt der vorliegenden Offenbarung die Aufgabe zugrunde, eine medizinische Handhabungsvorrichtung sowie ein Verfahren zur Steuerung einer Handhabungsvorrichtung anzugeben, die eine intuitive und fehlerarme Steuerung einer Mehrzahl von Funktionen ermöglichen. Vorzugsweise kann mit einer überschaubaren Anzahl an Eingabegeräten bzw. Eingabemöglichkeiten eine Mehrzahl unterschiedlicher Funktionen gesteuert werden. Vorzugweise kann dies derart erfolgen, dass es keine nachteiligen Wechselwirkungen/Überschneidungen gibt. Die Bedienung ist vorzugsweise hinreichend eindeutig, um die Gefahr von Fehlbedienungen zu verringern.

Vorzugsweise ist die Handhabungsvorrichtung derart gestaltet, dass bei der Bedienung/Steuerung keine übermäßige Ablenkung von der eigentlichen Tätigkeit gegeben ist.

Ferner soll im Rahmen der vorliegenden Offenbarung eine Handhabungsvorrichtung angegeben werden, welche die Arbeitsbedingungen für den Benutzer/Operateur optimiert und dazu beiträgt, dass der Benutzer die Übersicht behält.

Gemäß einem ersten Aspekt der vorliegenden Offenbarung wird diese Aufgabe durch eine medizinische Handhabungsvorrichtung gelöst, die Folgendes aufweist:
- einen Instrumentenhalter zur Aufnahme eines Beobachtungsinstruments mit einem Bildaufnehmer zur Erfassung eines Bildausschnitts,
- eine robotische Handhabungseinheit, die den Instrumentenhalter trägt,
- eine Steuereinrichtung mit einer Instrumentensteuereinheit zur Steuerung des Beobachtungsinstruments und einer Handhabungssteuereinheit zur Steuerung der robotischen Handhabungseinheit, und
- ein mit der Steuereinrichtung gekoppeltes Eingabegerät zur Wahl eines wiederzugebenden Bildausschnitts,
wobei die Steuereinrichtung dazu ausgebildet ist, die robotische Handhabungseinheit in Reaktion auf Bedienereingaben am Eingabegerät zu steuern, um den erfassten Bildausschnitt zu ändern, und
wobei die Steuereinrichtung dazu ausgebildet ist, Richtungsbefehle am Eingabegerät in Abhängigkeit von einer gegebenen Orientierung des Bildaufnehmers in Bewegungsvorgaben zu überführen, insbesondere in Bewegungsvorgaben für die robotische Handhabungseinheit.

Die der Offenbarung zugrunde liegende Aufgabe wird auf diese Weise vollkommen gelöst.

Erfindungsgemäß wird nämlich durch die Ausgestaltung der Steuereinrichtung sichergestellt, dass der Bildausschnitt intuitiv vom Bediener verlagert werden kann, und zwar unabhängig von der (äußeren) Ausrichtung des Beobachtungsinstruments. Dies hat den Vorteil, dass das Beobachtungsinstrument und die robotische Handhabungseinheit günstig in Relation zum Patienten positionierbar sind, so dass das übrige Sichtfeld nicht oder nur wenig gestört ist.

Die robotische Handhabungseinheit kann auch als telemedizinische Handhabungseinheit bezeichnet werden. Auch wenn es grundsätzlich vorstellbar ist, die Handhabungseinheit voll- oder teilautomatisiert zu betreiben, so ist jedoch zumindest in beispielhaften Ausführungsformen eine Steuerung durch den Bediener/Operateur vorgesehen.

Diese Funktion kann genutzt werden, um den gewählten Bildausschnitt zu verschieben, insbesondere in einer gedachten Ebene. Über das Eingabegerät können Steuerbefehle in Form von Richtungsbefehlen eingegeben werden, die anschließend durch die Steuereinrichtung umgesetzt werden, so dass die Handhabungseinheit unter Berücksichtigung der konkreten Ausrichtung des Bildaufnehmers angesteuert wird. Der Benutzer kann das Bild intuitiv verschieben, indem Richtungsbefehle am Eingabegerät wie etwa rechts, links, vorne und hinten in korrespondierende Verschiebungen/Bewegungen des angezeigten Bildausschnitts überführt werden. Dies vereinfacht die Bedienung deutlich. Die Gefahr von Fehlbedienungen, welche gerade im medizinischen Umfeld große Auswirkungen haben können, kann verringert werden. Die Bewegungsvorgaben werden schlussendlich in Steuerbefehle für Antriebe der Achsen der robotischen Handhabungseinheit überführt.

Eine Bewegungseingabe am Eingabegerät hat üblicherweise eine Richtungsinformation und eine Weginformation (Betrag). Bei den Bewegungsvorgaben handelt es sich um Steuersignale zur Steuerung der robotischen Handhabungseinheit. Der Bildausschnitt ist derjenige Teil eines Motivs, welcher zur Anzeige ausgewählt ist. Der Bildausschnitt umfasst in beispielhaften Ausgestaltungen eine Untermenge eines Aufnahmebereichs des Bildaufnehmers.

Generell kann das Eingabegerät dazu genutzt werden, um über Bedienereingaben den angezeigten Bildausschnitts zu manipulieren. Dies kann eine Bewegung (Verschiebung) umfassen. Vorstellbar ist jedoch auch eine Drehung (Rotation) sowie eine Vergrößerung/Verkleinerung (Zoom bzw. Veränderung des Abbildungsmaßstabs). Ferner ist es vorstellbar, über das Eingabegerät einen Fokus-Antrieb zur Verstellung einer Fokusebene (Schärfeebene) anzusteuern.

Das Eingabegerät ist zur Steuerung des Beobachtungsinstruments aber auch zur Steuerung der robotischen Handhabungseinheit nutzbar. Dies vereinfacht die Bedienung für den Bediener deutlich. Es ist nicht unbedingt nötig, zwei separate Eingabegeräte zu nutzen.

Es ist auch vorstellbar, andere Instrumente als Beobachtungsinstrumente am Instrumentenhalter der Handhabungseinheit aufzunehmen. Demgemäß umfasst der Begriff Instrumente grundsätzlich Beobachtungsinstrumente, aber auch chirurgische Instrumente wie Zangen, Scheren, Pinzetten, Sauger, etc.

Die Bewegung des Bildausschnitts kann einerseits über die robotische Handhabungseinheit bewirkt werden, indem diese das Beobachtungsinstrument mit dem Bildaufnehmer tatsächlich bewegt. Es ist jedoch auch vorstellbar, den Bildausschnitt digital zu verschieben. Dies ist etwa dann möglich, wenn ein Bildaufnehmer genutzt wird, dessen Aufnahmebereich größer als der aktuell gewählte Bildausschnitt ist. In einem solchen Fall kann der Aufnahmebereich innerhalb des gewählten Bildausschnitts verschoben werden.

Die Instrumentensteuereinheit kann als CCU/Controller/Konsole bezeichnet werden. Das Eingabegerät für die Manipulation (Bewegung) des Bildausschnitts ist in beispielhaften Ausgestaltungen über eine Schnittstelle an die Instrumentensteuereinheit angekoppelt. Dies heißt mit anderen Worten, Steuerbefehle für die Handhabungssteuereinheit werden (signaltechnisch) vom Eingabegerät über die Instrumentensteuereinheit an die Handhabungseinheit übergeben. Ferner kann der Status der Bildeinstellung (Zoom, Focus, ROI Position und Bildorientierung) der zu bewegenden Einheit (z.B. Exoskop) über die CCU an die Handhabungseinheit weitergegeben werden. Die Handhabungseinheit kann dann entsprechend darauf reagieren, beispielsweise durch Änderung der Bewegungsrichtung bei geänderter Orientierungseinstellung, oder durch Änderung des Pivotierungsradius bei geänderter Fokuseinstellung.

Die Steuereinrichtung kann verteilt sein und demgemäß eine Instrumentensteuereinheit zur Steuerung des Beobachtungsinstruments und eine separate Handhabungssteuereinheit zur Steuerung der robotischen Handhabungseinheit umfassen, die miteinander kommunizieren. Es versteht sich, dass die verteilte Gestaltung auch virtuell (softwaremäßig) realisiert werden kann. Gleichwohl ist zumindest in beispielhaften Ausführungsformen eine hardwaremäßige Trennung von Instrumentensteuereinheit und Handhabungssteuereinheit vorstellbar. Auf diese Weise bleibt die Instrumentensteuereinheit (CCU/Konsole) universell nutzbar, also auch für handgehaltene/handgeführte Instrumente.

Das Eingabegerät ist in einer beispielhaften Ausgestaltung mit der Instrumentensteuerungseinheit verbunden, wobei die Instrumentensteuerungseinheit - signaltechnisch - zwischen dem Eingabegerät und der Handhabungssteuereinheit angeordnet ist, also diesen zwischengeordnet. Ferner ist in dieser Ausgestaltung die Instrumentensteuereinheit - signaltechnisch - zwischen dem Beobachtungsinstrument und dem Eingabegerät angeordnet. Signale werden über die Instrumentensteuereinheit weitergeleitet bzw. gegebenenfalls sogar durchgeschliffen.

In einer beispielhaften Ausgestaltung ist ferner vorgesehen, dass das Eingabegerät (für den Fall der Steuerung eines Bildaufnahmeparameters direkt am Beobachtungsinstrument) signaltechnisch über die robotische Handhabungseinheit mit dem Beobachtungsinstrument verbindbar ist.

Ein Aspekt der vorliegenden Offenbarung beruht darauf, dass die Handhabungssteuereinheit die robotische Handhabungseinheit in Abhängigkeit von Parametern des Beobachtungsinstruments steuert. Dies kann insbesondere Parameter eines Beobachtungskopfes/Kamerakopfes des Beobachtungsinstruments betreffen. Beispielsweise kann die Handhabungssteuereinheit die Geschwindigkeit der Bewegung der Glieder der robotischen Handhabungseinheit in Abhängigkeit von einer gegebenen Vergrößerungsstufe (Zoom-Faktor, Fokusabstand bzw. Objektabstand). So kann etwa bei einem großen Zoom-Faktor bzw. einem kleinen Objektabstand (entsprechend einer Detaildarstellung) die Verfahrgeschwindigkeit der robotischen Handhabungseinheit verringert werden. Demgemäß kann etwa bei einem kleinen Zoom-Faktor bzw. einem großen Objektabstand (entsprechend einer Übersichtsdarstellung) die Verfahrgeschwindigkeit der robotischen Handhabungseinheit erhöht werden.

In einer weiteren beispielhaften Ausgestaltung weist die Handhabungsvorrichtung ferner eine Wiedergabeeinheit zur Anzeige des erfassten Bildausschnitts auf, wobei die Steuereinrichtung die gegebene Orientierung des Bildaufnehmers bei der Ansteuerung der Wiedergabeeinheit zur Wiedergabe des Bildausschnitts berücksichtigt. Mit anderen Worten gibt es in einer beispielhaften Ausführungsform einen künstlichen Horizont bzw. ein spezifisches Koordinatensystem für den Bildaufnehmer. Dieser künstliche Horizont bzw. dieses spezifische Koordinatensystem definiert die Lage des angezeigten Bildausschnitts am Anzeigegerät. Das Anzeigegerät ist etwa ein Monitor, ein Bildschirm, ein Projektor, eine spezifische Brille zur Anzeige (HMD - Head-Mounted Display) oder dergleichen.

Insbesondere bei Stereo-Bildaufnehmern für die stereoskopische Darstellung oder sogar für die 3D-Darstellung ist die Ausrichtung von hoher Bedeutung, damit der gewünschte Versatz zwischen dem rechten und dem linken Bildsensor in der richtigen Ebene angeordnet ist. Aus diesem Grund kann auch ein stereoskopischer Bildausschnitt nicht beliebig und kontinuierlich softwaremäßig rotiert werden. Stattdessen ist in einem solchen Fall regelmäßig eine Hardware-Rotation zur Ausrichtung des Horizonts erforderlich.

Gemäß einer weiteren beispielhaften Ausgestaltung der Handhabungsvorrichtung ist die Steuereinrichtung dazu ausgebildet, eine gegebene Ausrichtung des Bildaufnehmers zu erfassen und in Abhängigkeit davon eine Zuordnung zwischen einem Eingabegerät-Koordinatensystem und einem Koordinatensystem der Handhabungseinheit vorzunehmen, das die Orientierung des Bildaufnehmers widerspiegelt. Demgemäß beschreibt die Orientierung den Horizont bzw. die Drehlage des Bildaufnehmers.

Mit anderen Worten ist es zumindest in einigen Ausführungsbeispielen vorstellbar, dass eine Rechts-Links-Achse des Eingabegerätes eine Rechts-Links-Bewegung des angezeigten Bildausschnitts bewirkt. Gleichermaßen kann eine Vor-Zurück- Achse des Eingabegerätes eine Vor-Zurück-Bewegung bzw. Hoch-Runter-Bewegung des angezeigten Bildausschnitts bewirken. Die Information betreffend die aktuelle Orientierung des Bildaufnehmers wird beispielsweise vom Beobachtungsinstrument an die Steuereinrichtung übertragen.

Die Steuerung der robotischen Handhabungseinheit erfolgt nun unter der Prämisse, dass die Orientierung des Bildaufnehmers gewahrt wird. Eine entsprechende Interpolation der Bewegung über verschiedene Achsen der Handhabungseinheit trägt dazu bei, dass diese Zuordnung beibehalten wird.

Gemäß einer weiteren beispielhaften Ausgestaltung der Handhabungsvorrichtung weist das Beobachtungsinstrument einen Orientierungssensor zur Erfassung der Orientierung des Bildaufnehmers auf. Auf diese Weise kann der künstliche Horizont bzw. das dem Bildaufnehmer eigene Koordinatensystem signaltechnisch erfasst werden.

In einer weiteren beispielhaften Ausgestaltung erfolgt die Erfassung der gegebenen Orientierung des Bildaufnehmers mittelbar über die Anzeige, in dem anhand des angezeigten Bildausschnitts die gewünschte Orientierung (Horizontlage) definiert wird. Es ist grundsätzlich auch vorstellbar, die Orientierung des Bildaufnehmers mittelbar über eine Steuerung eines Antriebs zur Rotation/Verdrehung des Bildaufnehmers zu erfassen. Demgemäß wird die Orientierung nicht über einen Sensor erfasst sondern von den Soll-Vorgaben für den Antrieb hergeleitet.

Insbesondere bei einem Bildaufnehmer mit einem Beobachtungskanal (Mono-Bildaufnehmer) ist auch eine rein digitale Drehung und Erfassung des Horizonts vorstellbar.

Gemäß einer weiteren beispielhaften Ausgestaltung der Handhabungsvorrichtung ist der Bildaufnehmer im Beobachtungsinstrument drehbar. Dies bezieht sich insbesondere auf eine Achse senkrecht zur Bildebene des Bildaufnehmers. Beispielhafte Ausführungsformen mit Stereo-Bildaufnehmer nutzen eine solche Funktion. Es ist grundsätzlich vorstellbar, den Bildaufnehmer manuell verdrehbar zu gestalten. Es ist jedoch auch vorstellbar, einen Drehantrieb/Rotationsantrieb für den Bildaufnehmer vorzusehen. Die Verdrehbarkeit des Bildaufnehmers ermöglicht eine Bildaufrichtung.

Gemäß einer weiteren beispielhaften Ausgestaltung der Handhabungsvorrichtung ist die Steuereinrichtung dazu ausgebildet, den durch den Bildaufnehmer erfassten Bildausschnitt digital zu rotieren. Dies ist insbesondere bei einem Mono-Bildaufnehmer mit nur einem Beobachtungskanal vorstellbar.

Gemäß einer weiteren beispielhaften Ausgestaltung der Handhabungsvorrichtung weist das Beobachtungsinstrument einen Stereo-Bildaufnehmer mit zumindest zwei Bildsensoren auf. In einem solchen Fall ist die Bildaufrichtung über die Verdrehbarkeit des Bildaufnehmers von Vorteil. Auf diese Weise kann die Ausrichtung der Beobachtungskanäle mit der menschlichen Augenpartie bewerkstelligt werden. Es sind auch Ausführungsformen mit mehr als zwei Bildsensoren vorstellbar, etwa wenn jeweils zwei Sensoren einem Spektralbereich zugeordnet sind (sichtbares Licht, Infrarot, etc.).

Gemäß einer weiteren beispielhaften Ausgestaltung der Handhabungsvorrichtung ist die Steuereinrichtung dazu ausgebildet, eine Zuordnung zwischen der Orientierung des Bildaufnehmers und Bewegungsachsen für die Eingabe am Eingabegerät derart vorzunehmen, dass Bewegungsrichtungen des durch die Wiedergabeeinheit angezeigten Bildausschnitts mit Richtungsvorgaben am Eingabegerät in Übereinstimmung gebracht werden. Auf diese Weise ist es für den Bediener nicht erforderlich, eine gedankliche Ausrichtung zwischen den unterschiedlichen Koordinatensystemen/Orientierungen vorzunehmen. Der Bediener kann sich primär am angezeigten Bildausschnitt orientieren, um diesen in gewünschter Weise zu bewegen.

Demgemäß umfasst die Umsetzung der Bedienbefehle (Richtungsbefehle und Verfahrbefehle) durch die Steuereinrichtung eine Koordinatentransformation, die bei der Ansteuerung der Handhabungseinheit berücksichtigt wird. Die Bewegungsachsen entsprechen beispielhaft entsprechenden Bewegungsfreiheitsgraden (vor, zurück, rechts, links, etc.).

Gemäß einer weiteren beispielhaften Ausgestaltung der Handhabungsvorrichtung ist die Steuereinrichtung dazu ausgebildet, die Bewegungsvorgaben in Steuerbefehle für Bewegungsachsen der robotischen Handhabungseinheit zu überführen. Auf diese Weise kann auch eine mehrachsige Handhabungseinheit in einfacher Weise durch Bedienung am Eingabegerät gesteuert werden.

Gemäß einer weiteren beispielhaften Ausgestaltung der Handhabungsvorrichtung umfasst die robotische Handhabungseinheit eine mehrgliedrige Kinematik mit einer Mehrzahl von Koppelgliedern, welche von der Handhabungssteuereinheit der Steuereinrichtung gesteuert werden. Somit kann das Beobachtungsinstrument im gegebenen Raum mit großer Bewegungsfreiheit kontrolliert verfahren werden. Es handelt sich bei der mehrgliedrigen Kinematik beispielsweise um eine serielle Kinematik. Es versteht sich, dass auch eine parallele oder gemischt seriell-parallele Kinematik verwendbar ist.

Gemäß einer weiteren beispielhaften Ausgestaltung der Handhabungsvorrichtung ist das Eingabegerät als Mehrachs-Eingabegerät gestaltet, vorzugsweise als Einhand-Mehrachs-Eingabegerät, wobei das Eingabegerät Bedienbewegungen in Form von Schubbewegungen oder Schwenkbewegungen in zumindest zwei Achsen erlaubt, um Bewegungssignale zur zweidimensionalen Bewegung des Bildausschnitts in einer Ebene zu erfassen. Das Eingabegerät kann etwa als sogenannte 3D-Maus gestaltet sein. Durch geeignete Einwirkung auf das Eingabegerät, insbesondere auf ein Betätigungselement des Eingabegerätes, kann eine Bewegung des Bildausschnitts in der Ebene, insbesondere in einer Objektebene bzw. dem Aufnahmebereich gesteuert werden.

Es ist also vorstellbar, beim Eingabegerät ein Eingabeelement vorzusehen, welches in verschiedenen Achsen bewegbar ist, wobei die Bewegungen (beispielsweise Translationsbewegung entlang zweier oder mehr Achsen, sowie Rotationsbewegung bzw. Schwenkbewegung entlang zweier oder mehr Achsen) über geeignete Sensoren erfasst und in Steuerbefehle überführt werden.

Gemäß einer weiteren beispielhaften Ausgestaltung der Handhabungsvorrichtung richtet die Steuereinrichtung die zwei Bewegungsachsen des Eingabegerätes mit der gegebenen Orientierung des Bildaufnehmers aus, so dass Bedienbewegungen eines Eingabeelements des Eingabegerätes in richtungsgleichen Bewegungen des angezeigten Bildausschnitts resultieren. Dies sorgt dafür, dass der Bediener intuitiv über Bewegungen nach links, rechts, oben/vorne oder unten/hinten die gewünschten Bewegungen steuern kann. Der Bediener muss sich keine Gedanken um die Koordinatentransformation machen. Dies wird durch die Steuereinrichtung bewerkstelligt.

Gemäß einer weiteren beispielhaften Ausgestaltung der Handhabungsvorrichtung ist das Eingabegerät als Einhand-Eingabegerät gestaltet, wobei das Eingabegerät Bedienbewegungen zumindest in Form einer Rotation um eine Längsachse oder einer Translation entlang der Längsachse erfasst, um Bewegungssignale zur Steuerung einer Zoom-Funktion sowie für eine Fokuseinstellung zu erfassen. Demgemäß kann das Eingabegerät weitere Funktionen erfüllen. In einfacher Weise kann in nur einem Betriebsmodus eine Mehrzahl von Funktionen (Bewegung, Vergrößerung, etc.) gesteuert werden. Es ist jedoch auch vorstellbar, verschiedene Betriebsmodi vorzusehen, um eine eindeutige Steuerung zu erlauben.

Wie vorstehend bereits ausgeführt, kann ein Zoom-Modus einerseits einen Digital-Zoom umfassen. Es ist jedoch auch vorstellbar, die Zoom-Funktion (genauer: Änderung des Abbildungsmaßstabs) durch Änderung des Objektabstands zwischen Beobachtungsinstrument und Objektebene zu bewerkstelligen (optischer Zoom). Mit anderen Worten kann der Bildaufnehmer näher an das zu beobachtende Objekt herangefahren werden. Wenn der Objektabstand verändert wird, ist es in zumindest einigen Ausführungsformen erforderlich, auch die Scharfstellung/Fokussierung anzupassen. Dies erfolgt beispielsweise über den Fokus-Antrieb.

In einer beispielhaften Ausführungsform weist das Eingabegerät ein Eingabeelement auf, wobei eine Hubachse vorgesehen ist, entlang der das Eingabeelement in zwei Richtungen bewegbar bzw. mit Kraft beaufschlagbar ist. Zusätzlich ist es vorstellbar, das Eingabeelement um die Hubachse drehbar zu gestalten. Auf diese Weise kann etwa eine Zoom-Funktion über Drücken und Ziehen am Eingabeelement entlang der Hubachse bewerkstelligt werden. Ferner ist es vorstellbar, den Fokus-Antrieb über ein Drehen des Eingabeelements zu steuern. Es ist jedoch umgedreht auch vorstellbar, den Fokusantrieb über die Hubbewegung und den "Zoom-Antrieb" (Veränderung des Abbildungsmaßstabs) über die Drehbewegung zu steuern.

Erneut wird angemerkt, dass ein Wechsel zwischen der Detaildarstellung und der Übersichtsdarstellung durch einen sogenannten Digital-Zoom und ergänzend hierzu durch eine Veränderung des Arbeitsabstands zwischen dem Beobachtungsinstrument und der Objektebene über die robotische Handhabungseinheit herbeigeführt werden kann. In beispielhaften Ausführungsformen sind beide Modi vorgesehen, wobei die Steuereinrichtung dazu ausgestaltet ist, einen sanften "Übergang" zwischen beiden Modi zu ermöglichen. Idealerweise merkt der Bediener gar nicht, ob ein Digitalzoom oder eine Abbildungsmaßstab durch Veränderung des Objektabstands (Arbeitsabstands) vorliegt wird. Gleichwohl wird häufig vereinfachend von einer Zoom-Funktion gesprochen.

Es versteht sich, dass auch alternative Ausführungsformen vorstellbar sind, bei denen das Beobachtungsinstrument einen optischen Zoom aufweist, also ein Objektiv mit variabler Brennweite.

Gemäß einer weiteren beispielhaften Ausgestaltung der Handhabungsvorrichtung ist die Handhabungssteuereinheit der Steuereinrichtung dazu ausgestaltet, den angezeigten Bildausschnitt in Reaktion auf Bedienbefehle am Eingabegerät über eine Bewegung der robotischen Handhabungseinheit zu bewegen, und wobei die Instrumentensteuereinheit der Steuereinrichtung dazu ausgestaltet ist, den angezeigten Bildausschnitt in Reaktion auf Bedienbefehle am Eingabegerät über eine digitale Verschiebung des angezeigten Bildausschnitts in einem erfassten Aufnahmebereich zu bewegen. Auf diese Weise kann der (elektronische) Digital-Zoom und der Zoom (genauer: Änderung des Abbildungsmaßstab) über eine Bewegung des gesamten Beobachtungsinstruments über die Handhabungseinheit kombiniert werden.

Der Bildausschnitt kann also in beispielhaften Ausgestaltungen über die Handhabungseinheit und alternativ über eine digitale Bildverschiebung bewegt werden. Letzteres ist dann der Fall, wenn der angezeigte Bildausschnitt kleiner als der Aufnahmebereich ist. Beide Funktionen können kombiniert werden.

Gemäß einer weiteren beispielhaften Ausgestaltung der Handhabungsvorrichtung ist das Eingabegerät in einem ersten Betriebsmodus zur Steuerung des Instruments und in einem zweiten Betriebsmodus zur Steuerung der robotischen Handhabungseinheit betreibbar, wobei die Handhabungsvorrichtung ferner einen Freigabeschalter zur Aktivierung des zweiten Betriebsmodus aufweist, in dem die robotische Handhabungseinheit in Reaktion auf Eingabebefehle am Eingabegerät verfahrbar ist.

Auf diese Weise wird eine Trennung zwischen Funktionen, bei denen die Handhabungseinheit womöglich oder auf jeden Fall bewegt wird, und Funktionen, bei denen die Handhabungseinheit nicht bewegt wird, möglich. Dies erhöht die Sicherheit. Dies gilt insbesondere dann, wenn der Bediener die Handhabungseinheit mittelbar steuert und sich dabei am angezeigten Bild und nicht unbedingt an den konkreten Bewegungen von Elementen der Handhabungseinheit und dem Beobachtungsinstrument orientiert.

Demgemäß ist es in verschiedenen Ausführungsform vorstellbar, einen Freigabeschalter für Manipulationen des Bildausschnitts unter Nutzung der robotischen Handhabungseinheit vorzusehen, und zwar insbesondere für den Fall, dass eine Bewegung des Bildausschnitts über die robotische Handhabungseinheit erfolgt. Auf diese Weise wird sichergestellt, dass die Handhabungseinheit nicht unbeabsichtigt bewegt wird.

Gemäß einer weiteren beispielhaften Ausgestaltung der Handhabungsvorrichtung ist die Steuereinrichtung dazu ausgestaltet, eine Initialisierungsprozedur durchzuführen, um Konfigurationsinformationen betreffend das gehaltene Beobachtungsinstrument zu erfassen, wobei die Initialisierung vorzugsweise eine Abfrage über die Instrumentensteuereinheit aufweist, und wobei die Konfigurationsinformationen an die Handhabungssteuereinheit übermittelt und bei der Steuerung der Handhabungseinheit berücksichtigt werden.

Auf diese Weise kann die Steuereinrichtung beispielsweise ermitteln, welche Art von Beobachtungsinstrument momentan an der robotischen Handhabungseinheit befestigt ist. Die Art des Beobachtungsinstruments betrifft beispielsweise dessen Abmessungen, Parameter von dessen Bildaufnehmer, die Fähigkeit zum Datenaustausch, eine etwaige Rotationsposition zum Drehen des Bildaufnehmers, und einen etwaigen Sensor zur Erfassung der Drehlage des Bildaufnehmers, usw.

Der Begriff Initialisierungsprozedur ist nicht so zu verstehen, dass es sich lediglich um eine einmalige Prozedur handelt. Die Initialisierungsprozedur kann wiederholt ausgeführt werden, etwa bei jeder konkreten Behandlung oder Diagnoseausgabe, also beispielsweise beim Hochfahren der Steuereinrichtung , oder bei jeder Umrüstung der Handhabungsvorrichtung. Die Initialisierungsprozedur auch kann gezielt und automatisiert wiederholt werden, etwa durch periodische Wiederholungen. Umgekehrt ist es vorstellbar, dass der Bediener die Initialisierungsprozedur bewusst auslösen kann.

Mit anderen Worten kann während der Initialisierungsprozedur eine Art Offset bestimmt werden, wobei die Steuereinrichtung (Handhabungssteuereinheit) für die robotische Handhabungseinheit diesen Offset bei der Steuerung der robotischen Handhabungseinheit nutzt. Dieser Offset definiert beispielsweise die Lage des Bildaufnehmers in Relation zu den Elementen der Handhabungseinheit. Der Offset kann die geometrische Gestalt/Ausdehnung/Orientierung des Beobachtungsinstruments beschreiben. Auf diese Weise kann der Bildaufnehmer des Beobachtungsinstruments genau gesteuert werden, um den Bildausschnitt wie gewünscht zu verfahren.

Beispielhaft ist es vorstellbar, die der Initialisierungsprozedur innewohnende Abfrage über eine Betätigung des Freigabeschalters zu starten. Die Initialisierungsprozedur kann auch als Setup-Prozedur bezeichnet werden. Demgemäß ist es vorstellbar, verschiedene Kamerasysteme/Beobachtungsinstrumente zu nutzen. Die Daten (Konfigurationsinformationen) können durch das Beobachtungsinstrument direkt bereitgestellt werden. Alternativ kann das Beobachtungsinstrument über seine ID erkannt werden, wobei anhand der ID Daten aus einer Datenbank abzufragen sind.

Gemäß einer weiteren beispielhaften Ausgestaltung der Handhabungsvorrichtung ist die Steuereinrichtung dazu ausgestaltet, den angezeigten Bildausschnitt bedarfsweise zu spiegeln, wobei die Umsetzung von Bedienbefehlen am Eingabegerät die Spiegelung berücksichtigt. Auf diese Weise kann etwa ein Flip-Modus bereitgestellt werden.

Eine Spiegelung erfolgt beispielsweise um eine horizontale oder vertikale Achse. Etwas Derartiges kann beispielsweise dann erfolgen, wenn ein anderer Bediener die Steuerung der Handhabungseinheit übernimmt, der aus Sicht des zuvor aktiven Bedieners auf einer gegenüberliegenden Seite des Patienten steht.

Gemäß einer weiteren beispielhaften Ausgestaltung der Handhabungsvorrichtung ist die Steuereinrichtung dazu ausgestaltet, die Handhabungseinheit derart anzusteuern, dass das Beobachtungsinstrument durch interpolierte Bewegung der Handhabungseinheit um eine virtuelle Kippachse verschwenkbar ist, die parallel zum Bildaufnehmer angeordnet ist. Auf diese Weise kann ein Instrument mit variabler Blickrichtung "simuliert" werden. Instrumente ohne integrierten Schwenkantrieb können somit auch einen solchen Freiheitsgrad bzw. eine solche Funktion bereitstellen. Es versteht sich, dass diese Funktion insbesondere bei Instrumenten vorstellbar ist, welche außerhalb des Körpers des Patienten angeordnet sind.

Es ist vorstellbar, dass in alternativen Ausführungsformen Instrumente mit variabler Blickrichtung und entsprechenden (internen) Antrieben vorgesehen sind, wobei ebenso die Steuerung der Antriebe über das Eingabegerät erfolgt. Auf diese Weise kann eine intuitive Steuerung des Schwenkantriebs erfolgen.

Gemäß einer weiteren beispielhaften Ausgestaltung der Handhabungsvorrichtung ist die Steuereinrichtung dazu ausgestaltet, die robotische Handhabungseinheit in einem Direktsteuermodus zu betreiben, um das Beobachtungsinstrument im Raum zu bewegen und auszurichten, wobei Bedienbefehle an der robotischen Handhabungseinheit durch Einwirkung auf ein dem Instrument benachbartes Element der Handhabungseinheit erzeugbar sind, und wobei die Handhabungssteuereinheit dazu ausgestaltet ist, die robotische Handhabungseinheit derart anzusteuern, dass das Beobachtungsinstrument der induzierten Bewegung folgt, wobei die Bedienbefehle im Direktsteuermodus vorzugsweise über ein Bedienelement abgegeben werden, welches über einen Sensor ein Freigabesignal für den Direktsteuermodus erzeugt.

Ein solcher Direktsteuermodus ("Direct Drag Mode") kann insbesondere für die Grobpositionierung des Beobachtungsinstruments in Bezug auf das Objektfeld genutzt werden. Sofern die robotische Handhabungseinheit in geeigneter Weise angesteuert wird, ergibt sich eine quasi-manuelle Verstellung direkt am Instrumentenhalter oder zumindest in der Nachbarschaft des Beobachtungsinstruments.

In einem solchen Modus kann folglich der Instrumentenhalter mit dem daran aufgenommenen Beobachtungsinstrument quasi-manuell im Raum bewegt und ausgerichtet werden, wobei die Steuereinrichtung dazu ausgebildet ist, die robotische Handhabungseinheit jeweils selbsttätig in der aktuellen Position/Orientierung zu halten, aber eine manuelle Bewegung durch direktes Greifen und Bewegen zu ermöglichen

Es ist vorstellbar, die Antriebe der Elemente/Glieder der kinematischen Kette der robotischen Handhabungseinheit im Direktsteuermodus derart zu überwachen, dass die Bedienbefehle erfasst werden. Somit kann besagte "Folgebewegung" erzeugt werden. Mit anderen Worten dient die robotische Handhabungseinheit im Direktsteuermodus selbst als Eingabegerät.

In diesem Modus ist es aus Sicht der robotischen Handhabungseinheit nicht unbedingt notwendig, Informationen betreffend das Beobachtungsinstrument umfangreich abzufragen, da ja die Bewegung direkt an der robotischen Handhabungseinheit manuell induziert und gesteuert wird.

In einer beispielhaften Weiterbildung dieser Ausgestaltung ist ein Eingabegerät mit einem Eingabeelement vorgesehen, um die Handhabungseinheit und damit das Beobachtungsinstrument im Direktsteuermodus zu steuern. Das Eingabegerät ist beispielhaft direkt an einem Element der Handhabungseinheit angeordnet und vorzugsweise dem Instrumentenhalter bzw. dem aufgenommenen Instrument benachbart. Das Eingabegerät bzw. dessen Eingabeelement selbst kann grundsätzlich einfach gehalten sein. Es kann sich hierbei um einen Griff handeln, über den der Bediener die Handhabungseinheit durch Ziehen, Drücken oder ähnlicher Weise bewegen/manipulieren kann.

In einer weiteren beispielhaften Ausgestaltung ist das Eingabegerät für den Direktsteuermodus mit einem Sensor versehen, der den Direktsteuermodus aktiviert. Ein solcher Sensor kann etwa eine Berührung oder Annäherung durch eine Hand des Bedieners erfassen. Auf diese Weise kann eine Freigabe des Direktsteuermodus erfolgen. In einem solchen Modus ist die Steuereinrichtung dazu ausgebildet, Bedienbewegungen des Bedieners zu erfassen und die Handhabungseinheit entsprechend nachzuführen.

Es ist vorstellbar, zu diesem Zweck Sensoren in den Bewegungsachsen der Handhabungseinheit vorzusehen. Es ist jedoch auch vorstellbar, Kennwerte/Parameter der Bewegungsachsen, also der Antriebsmotoren, zu überwachen, etwa Ströme und dergleichen, um die Steuersignale und folglich die von außen durch den Bediener induzierten Bewegungen detektieren zu können. Alternativ ist auch eine Sensorik vorstellbar, die nur die räumliche Position der vordersten Spitze der Handhabungseinheit bzw. das Beobachtungsinstrument selbst misst, z.B. optisches Trackingsystem.

Gemäß einem weiteren Aspekt wird die Aufgabe der vorliegenden Offenbarung durch ein Verfahren zur Steuerung einer Handhabungsvorrichtung gelöst, wobei die Handhabungsvorrichtung eine robotische Handhabungseinheit mit einem Instrumentenhalter und einem daran aufgenommenen Beobachtungsinstrument mit einem Bildaufnehmer zur Erfassung eines Bildausschnitts umfasst, wobei das Verfahren die folgenden Schritte aufweist:
- Bereitstellung des Beobachtungsinstruments am Instrumentenhalter,
- Erfassung von Steuerbefehlen zur Wahl eines wiederzugebenden Bildausschnitts über ein Eingabegerät, das mit einer Steuereinrichtung zur Steuerung des Beobachtungsinstruments und zur Steuerung der robotischen Handhabungseinheit gekoppelt ist, und
- Steuerung der robotischen Handhabungseinheit in Reaktion auf Bedienereingaben am Eingabegerät, um den erfassten Bildausschnitt zu ändern, umfassend:
   - Überführung von Richtungsbefehlen am Eingabegerät in Bewegungsvorgaben, insbesondere Bewegungsvorgaben für die robotische Handhabungseinheit, in Abhängigkeit von der gegebenen Orientierung des Bildaufnehmers.

Die Aufgabe der Offenbarung wird auch auf diese Weise vollständig gelöst.

Mit anderen Worten umfasst die Überführung der Richtungsbefehle eine Transformation zwischen einem Koordinatensystem bzw. einem "Horizont" des Eingabegerätes und einem Koordinatensystem bzw. einem "Horizont" des Beobachtungsinstruments, insbesondere von dessen Bildaufnehmer. Aktuelle Ausrichtungen des Eingabegerätes und des Beobachtungsinstruments (von dessen Bildaufnehmer) werden zu Steuerungszwecken in Übereinstimmung gebracht. Wenn diese Transformation systemseitig vorgenommen wird, erspart sich der Bediener eine gedankliche Transformation bei der Betätigung. Die Steuerung der Handhabungsvorrichtung zur Verschiebung des Bildausschnitts vereinfacht sich deutlich.

Die Änderung des Bildausschnitts kann eine Verschiebung des angezeigten Bildausschnitts zum Ziel haben. Dies kann durch geeignete Steuerung der Handhabungseinheit bewirkt werden. Es sind andere Manipulationen des Bildausschnitts vorstellbar, etwa eine Rotation, Verkippung, eine kombinierte Bewegung und/oder eine Vergrößerung/Verkleinerung.

Es ist vorstellbar, das Verfahren zur Steuerung einer medizinischen Handhabungsvorrichtung zu nutzen. Es ist vorstellbar, das Verfahren für chirurgische und/oder diagnostische Prozesse zu nutzen. Es ist jedoch auch vorstellbar, das Verfahren für andere als chirurgische und/oder diagnostische Prozesse zu nutzen. Es sind folglich auch Ausführungsbeispiele vorstellbar, in denen das Verfahren nicht zur Durchführung einer chirurgischen/diagnostischen Prozedur am menschlichen oder tierischen Körper genutzt wird.

Es versteht sich, dass das Steuerungsverfahren analog zu den beispielhaften Ausgestaltungen der Handhabungsvorrichtung weitergebildet sein kann und umgekehrt. Mit anderen Worten kann der Gegenstand beispielhafter Ausgestaltungen und Weiterbildungen, die auf die Handhabungsvorrichtung bezogen sind, auch zum Gegenstand entsprechende Ausgestaltungen des hier beschriebenen Verfahrens werden.

Es versteht sich, dass die vorstehend genannten und die nachstehend noch zu erläuternden Merkmale der Erfindung nicht nur in der jeweils angegebenen Kombination, sondern auch in anderen Kombinationen oder in Alleinstellung verwendbar sind, ohne den Rahmen der vorliegenden Erfindung zu verlassen.

Weitere Merkmale und Vorteile der Erfindung ergeben sich aus der nachfolgenden Beschreibung mehrerer bevorzugter Ausführungsbeispiele unter Bezugnahme auf die Zeichnungen. Es zeigen:
- Fig. 1: eine perspektivische Ansicht einer Ausführungsform einer medizinischen Handhabungsvorrichtung zur Beobachtung eines Objektfeldes bei einem Patienten;
- Fig. 2: eine weitere perspektivische Ansicht der Handhabungsvorrichtung gemäß Fig. 1 in einer abweichenden Ansichtsorientierung;
- Fig. 3: eine schematische, vereinfachte Teilansicht eines Bildaufnehmers bei einem Beobachtungskopf eines Beobachtungsinstruments sowie einer Wiedergabeeinheit zur Veranschaulichung einer Bildorientierung;
- Fig. 4: eine weitere Darstellung analog zur Fig. 3 mit einer korrigierten Bildorientierung durch Rotation des Bildaufnehmers;
- Fig. 5: eine perspektivische Teilansicht einer Ausführungsform einer Handhabungsvorrichtung mit einem Beobachtungsinstrument zur Veranschaulichung einer beispielhaften Funktion der Handhabungsvorrichtung;
- Fig. 6: eine weitere perspektivische Teilansicht einer Ausführungsform einer Handhabungsvorrichtung mit einem Beobachtungsinstrument zur Veranschaulichung einer weiteren beispielhaften Funktion;
- Fig. 7: eine weitere perspektivische Teilansicht einer Ausführungsform einer Handhabungsvorrichtung mit einem Beobachtungsinstrument zur Veranschaulichung einer weiteren beispielhaften Funktion;
- Fig. 8: eine schematische Ansicht einer Anordnung, umfassend zwei Eingabegeräte, welche bei der Handhabungsvorrichtung zu Steuerungszwecken nutzbar sind;
- Fig. 9: eine perspektivische, vereinfachte Teilansicht einer Ausführungsform einer Handhabungsvorrichtung mit einem Eingabeelement, das als Freigabeschalter fungiert;
- Fig. 10: eine weitere perspektivische Teilansicht einer Ausführungsform einer Handhabungsvorrichtung mit einem Beobachtungsinstrument zur Veranschaulichung einer weiteren beispielhaften Funktion;
- Fig. 11: eine Seitenansicht eines Instrumentenhalters mit einem daran aufnehmbaren Beobachtungsinstrument, in einem nicht montierten Zustand;
- Fig. 12: eine weitere Ansicht der Anordnung gemäß Fig. 12 im montierten Zustand;
- Fig. 13: ein schematisches Blockschaltbild zur Veranschaulichung einer Prinzipgestaltung einer Ausführungsform einer Handhabungsvorrichtung;
- Fig. 14: ein schematisch vereinfachtes Blockdiagramm zur Veranschaulichung einer Ausführungsform eines Verfahrens zur Steuerung einer Handhabungsvorrichtung; und
- Fig. 15: ein schematisch vereinfachtes Blockdiagramm zur Veranschaulichung einer Ausführungsform eines Verfahrens zur lagekorrigierten Steuerung einer Handhabungseinheit unter Berücksichtigung einer Orientierung eines Beobachtungsinstruments.

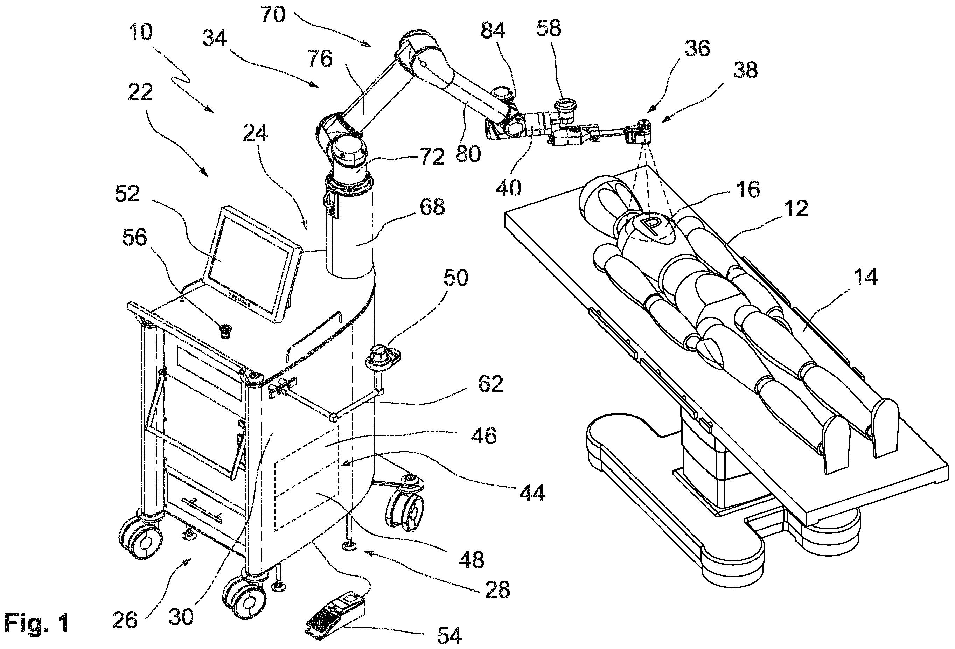

Fig. 1 veranschaulicht anhand einer perspektivischen Übersichtsdarstellung eine insgesamt mit 10 bezeichnete Handhabungsvorrichtung. Die Handhabungsvorrichtung 10 kann auch als medizinische Handhabungsvorrichtung bezeichnet werden. Die Handhabungsvorrichtung 10 ist in dem in Fig. 1 gezeigten Ausführungsbeispiel einem Patienten 12 zugeordnet, der auf einem Tisch 14 gelagert ist. Die Handhabungsvorrichtung 10 ist für therapeutische, chirurgische und/oder diagnostische Zwecke nutzbar. Es ist jedoch auch eine Verwendung für nicht-therapeutische, nichtchirurgische und/oder nicht-diagnostische Zwecke vorstellbar. Dies kann die Verwendung bei Übungen bzw. Simulationen umfassen.

In dem veranschaulichten Ausführungsbeispiel dient die Handhabungsvorrichtung 10 zur Beobachtung eines Objektfeldes 16. Bei dem Objektfeld 16 handelt es sich beispielhaft um einen Teil des Körpers des Patienten 12. Aus Veranschaulichungszwecken ist in zumindest einigen der hierin gezeigten Figuren das Objektfeldes 16 durch den Buchstaben P gekennzeichnet. Dies ist nicht einschränkend zu verstehen.

In dem in Fig. 1 gezeigten Ausführungsbeispiel ist das Objektfeld 16 außen am Körper des Patienten 12 angeordnet. Demgemäß dient die Handhabungsvorrichtung 10 in diesem Ausführungsbeispiel zur Beobachtung des Körpers von außerhalb des Körpers. Es sind alternative Ausführungsbeispiele vorstellbar, bei denen die Handhabungsvorrichtung 10 zur Beobachtung des Körperinneren nutzbar ist, also beispielsweise zur endoskopischen oder laparoskopischen Beobachtung.

Allgemein dient die Handlungsrichtung 10 zur optischen Beobachtung im Bereich des sichtbaren elektromagnetischen Spektrums bzw. in benachbarten Randbereichen. Es geht in wesentlichen Ausführungsbeispielen folglich um die Beobachtung unter Nutzung von Weißlicht, InfrarotStrahlung oder UV-Strahlung. Für das menschliche Auge sichtbare Licht (Weißlicht) liegt etwa in einem Spektralbereich zwischen 380 nm und 780 nm. Strahlung im Nah-Infrarot-Bereich liegt im Bereich von etwa 780 nm bis 1400 nm. Sogenanntes Nahes UV-Licht (auch bezeichnet als Schwarzlicht oder UV-A-Licht) liegt im Bereich von etwa 315 bis 380 nm. Sogenanntes mittleres UV-Licht (auch bezeichnet als UV-B-Licht) liegt im Bereich von etwa 280 nm bis 315 nm.

Die vorgenannten Bereiche können für die Weißlicht-Beobachtung sowie für PDD-Anwendungen (Photodynamische Diagnostik) und PDT-Anwendungen (Photodynamische Therapie) genutzt werden. Hiervon kann auch die Fluoreszenzbeobachtung umfasst sein. In diesem Zusammenhang ist auch Fluoreszenzbeobachtung unter Nutzung von Indocyaningrün (ICG) mit Fluoreszenz im Nahinfrarotbereich denkbar.

Die Handhabungsvorrichtung 10 umfasst eine Plattform 22, welche etwa als Fahrwagen oder Wagen 24 gestaltet ist. Dies ist nicht einschränkend zu verstehen. Gleichwohl ist es zumindest in beispielhaften Ausgestaltungen vorgesehen, dass die Plattform 22 verfahrbar ist. Dies erhöht die Flexibilität und Eignung für diverse Anwendungsfälle. Demgemäß ist die Plattform 22 beispielsweise als Wagen 24 mit einem Fahrgestell 26 gestaltet. In der in Fig. 1 gezeigten Ausführungsform umfasst der Wagen 24 neben dem Fahrgestell 26 auch eine sogenannte Abstützung 28 bzw. eine entsprechende Stützeinheit.

Die Abstützung 28 wird genutzt, um den Wagen 24 während des Betriebs der Handhabungsvorrichtung 10 gegen ungewolltes Verfahren zu schützen. Demgemäß kann die Abstützung 28 dazu dienen, den Wagen 24 aufzubocken. Alternativ oder zusätzlich ist es vorgesehen, Räder des Fahrgestells 26 zu blockieren, im Sinne einer Feststellbremse. Der Status des Wagens 24 (fahrbar oder aufgebockt/festgestellt) kann über geeignete Sensoren überwacht werden, um einen Betrieb der Handhabungsvorrichtung 10 eben nur dann freizugeben, wenn sichergestellt ist, dass der Wagen 24 nicht ungewollt verfahrbar ist. Es versteht sich, dass der Wagen 24 auch in anderer Weise verankert/fixiert werden kann, um einen sicheren Betrieb der Handhabungsvorrichtung 10 zu ermöglichen.

Ferner weist die Plattform 22 ein Gehäuse 30 auf, das Elemente/Einheiten der Handhabungsvorrichtung 10 beherbergt. Auf diese Weise ergibt sich eine kompakte, übersichtliche Gestaltung. Ferner vereinfacht sich die Reinigung der Handhabungsvorrichtung 10. Alternativ ist auch eine Gestaltung als Regal oder Regalwagen vorstellbar. In beispielhaften Ausgestaltungen sind wesentliche Steuereinheiten für die Handhabungsvorrichtung 10 im Gehäuse 30 des Fahrwagens 24 angeordnet. Dies führt dazu, dass die Plattform 22 mobil ist, so dass ein Einsatz an verschiedenen Orten bzw. in verschiedenen Räumen vorstellbar ist. Es versteht sich, dass die Plattform 22 bzw. der Fahrwagen 24 gleichwohl mit der Umwelt gekoppelt sind, etwa zu Zwecken der Energieversorgung, Signalversorgung und/oder Medienversorgung.

Die Plattform 22 bzw. der die Plattform bildende Fahrwagen 24 trägt eine Handhabungseinheit 34. In den veranschaulichten Ausführungsbeispielen ist die Handhabungseinheit 34 als motorische Handhabungseinheit, beispielsweise als robotische Handhabungseinheit gestaltet. Alternativ kann die Handhabungseinheit 34 als Telemanipulatoreinheit bezeichnet werden. Demgemäß bildet die Plattform 22 eine Basis für die Handhabungseinheit 34. Zumindest in beispielhaften Ausführungsformen sind Steuereinrichtungen für die Handhabungseinheit 34 an der Plattform 22 bzw. in deren Gehäuse 30 angeordnet.

Die Handhabungseinheit 34 ist dazu ausgebildet, ein Instrument 36 zu tragen/zu halten. Über die Handhabungseinheit 34 kann das Instrument 36 motorisch bewegt werden. Demgemäß kann die Handhabungseinheit 34 als Telemanipulator für das Instrument 36 bezeichnet werden. Bei dem Instrument 36 handelt sich beispielsweise um ein medizinisches Instrument. Zumindest in beispielhaften Ausgestaltungen ist das Instrument 36 als Beobachtungsinstrument 38 gestaltet. Bei den Beobachtungsinstrument 38 handelt es sich beispielhaft um ein Instrument zur Beobachtung des Patienten 12 von außerhalb des Körpers, also mit Abstand zum Körper des Patienten 12. Ein solches Beobachtungsinstrument 38 kann als Exoskop gestaltet und bezeichnet sein. Es ist jedoch auch vorstellbar, das Beobachtungsinstrument 38 als Instrument zur Beobachtung des Inneren des Körpers des Patienten 12 zu gestalten, also etwa als Laparoskop oder Endoskop.

Das Instrument 36 ist an einem Instrumentenhalter 40 aufgenommen. Vorzugsweise ist das Instrument 36 lösbar am Instrumentenhalter 40 aufgenommen. Mit anderen Worten kann das Instrument 36 grundsätzlich auch vom Instrumentenhalter und folglich von der Handhabungseinheit 34 gelöst werden. Somit ist es vorstellbar, das Instrument 36 in alternativen Anwendungen auch handgeführt/handgehalten zu betreiben. Nachfolgend wird insbesondere aus Veranschaulichungsgründen davon ausgegangen, dass das Instrument 36 als Beobachtungsinstrument 38 zur Beobachtung eines Objektfeldes 16 genutzt wird, insbesondere als medizinisches Beobachtungsinstrument 38 zur Beobachtung eines Objektfeldes 16 bei einem Patienten 12.

In Fig. 1 veranschaulicht das Bezugszeichen 44 anhand gestrichelter Linien eine Steuereinrichtung 44, die an der Plattform 22 aufgenommen ist. Beispielhaft weist die Plattform 22 einen Wagen 24 mit einem Regalgestell auf, das eine Einhausung in Form eines Gehäuses 30 umfasst. Demgemäß kann die Steuerrichtung 44 an jenem Regalgestell aufgenommen und gehalten sein.

Zumindest in beispielhaften Ausführungsformen weist die Steuereinrichtung 44 eine Handhabungssteuereinheit 46 und eine Instrumentensteuereinheit 48 auf. Bei der Handhabungssteuereinheit 46 und der Instrumentensteuereinheit 48 kann es sich um diskrete, grundsätzlich separate Steuereinheiten/Steuermodule handeln. Mit anderen Worten können mehrere Geräte miteinander kombiniert werden, um die Steuereinrichtung 44 zu bilden. Es ist jedoch auch vorstellbar, die Steuereinrichtung 44 derart zu gestalten, dass die Handhabungssteuereinheit 46 und die Instrumentensteuereinheit 48 zumindest teilweise gemeinsame Hardware/Rechentechnik nutzen. Mit anderen Worten ist es vorstellbar, die Steuereinheiten 46, 48 diskret und/oder integral zu gestalten. Mischformen sind denkbar.

Zur Steuerung der Handhabungsvorrichtung 10 und insbesondere zur Interaktion mit der Steuereinrichtung 44 sind diverse Eingabegeräte für einen Bediener (etwa einen Operateur oder Assistenten) vorgesehen. Beispielsweise ist ein Eingabegerät 50 vorgesehen, welches als Einhand-Eingabegerät gestaltet ist. Beispielhaft ist das Eingabegerät 50 als sogenannte 3D-Maus gestaltet, zumindest ähnlich einer 3D-Maus. Mit anderen Worten kann das Eingabegerät 50 dazu ausgebildet sein, Bedienereingaben und folglich Steuerbefehle in mehreren Raumachsen zu erfassen, wobei die Eingabe über lediglich eine Hand erfolgt, die an ein einziges Eingabeelement angreift. Vorzugsweise dient das Eingabegerät 50 sowohl zur Steuerung der robotischen Handhabungseinheit 34 als auch zur Steuerung des Beobachtungsinstruments 38 bzw. zur Steuerung einer Wiedergabe eines durch das Beobachtungsinstrument 38 aufgenommenen Bildes. In diesem Zusammenhang wird erneut auf die DE 10 2015 121 017 A1 verwiesen, die die Verwendung eines Einhand-Eingabegerätes zur Steuerung von Bildaufnahmeparametern und zur Steuerung von Bildwiedergabeparametern zeigt.

Ein weiteres Eingabegerät 52 ist als sogenannter Touch-Monitor, also als Berührbildschirm gestaltet. Demgemäß ist das Eingabegerät 52 für Auswahlentscheidungen, allgemeines Einstellungen und ähnliche Funktionen nutzbar. Es ist grundsätzlich auch möglich, die robotische Handhabungseinheit 34 über das Eingabegerät 52 zu steuern. Das als Touch-Monitor gestaltete Eingabegerät 52 kann etwa genutzt werden, um allgemeine Einstellungen betreffend das Instrument 36 (Beobachtungsinstrument 38) vorzunehmen. Dies umfasst beispielsweise auch die Auswahl des aktuellen Typs des Beobachtungsinstruments 38. Ferner können Betriebsparameter zum Betrieb des Beobachtungsinstruments 38 über das Eingabegerät 52 ausgewählt und/oder eingegeben werden.

Ein weiteres Eingabegerät 54 ist als beispielsweise Fußschalter gestaltet. Der Fußschalter kann durch den Bediener betätigt werden, ohne dass hierfür die Hände benötigt werden. Das als Fußschalter gestaltete Eingabegerät 54 kann insbesondere als Freigabeschalter verwendet werden. Die Gestaltung als Fußschalter ist nicht einschränkend zu verstehen.

Grundsätzlich ist das Eingabegerät 54 dazu vorgesehen, bestimmte Funktionen/Operationen bei Bedarf freizugeben, und zwar nur auf ausdrücklichen Befehl des Bedieners. Anders gesagt kann durch das Eingabegerät verhindert werden, dass bestimmte Funktionen unbewusst ausgelöst werden. Auf diese Weise kann insbesondere die robotische Handhabungseinheit 34 sicher bedient werden. Dies bezieht sich insbesondere auf Bewegungen des Instrumentenhalters 40 (mit dem daran aufgenommenen Instrument 36) in Bezug auf den Patienten 12 oder den Tisch 14. Solche Bewegungen sollen dann möglich sein, wenn ein zusätzliches Freigabesignal über das Eingabegerät 54 vorliegt. Ferner kann das als Freigabeschalter dienende Eingabegerät 54 mit einer Sicherheitssteuerung (Freigabesteuerung) gekoppelt sein.

Ferner zeigt die in Fig. 1 veranschaulichte Ausgestaltung der Handhabungsvorrichtung 10 ein weiteres Eingabegerät 56, welches als Knopf oder Taster gestaltet ist. Das Eingabegerät 56 kann grundsätzlich als Not-Aus-Taster gestaltet sein. Demgemäß kann das Eingabegerät 56 eine aktuelle Aktion der Handhabungsvorrichtung 10, insbesondere der robotischen Handhabungseinheit 34 abbrechen. Es ist jedoch auch vorstellbar, das Eingabegerät 56 ähnlich dem zuvor beschriebenen Eingabegerät 54 als Freigabeschalter zur Aktivierung (Ermöglichung) bestimmter Funktionen auszuführen. Beide Ausführungsformen erhöhen die Sicherheit beim Betrieb der Handhabungsvorrichtung 10.

Ein weiteres Eingabegerät 58 ist in der in Fig. 1 veranschaulichten beispielhaften Ausgestaltung der Handhabungsvorrichtung 10 direkt bei der robotischen Handhabungseinheit 34 vorgesehen. Das Eingabegerät 58 ist am oder nahe dem Instrumentenhalter 40 angeordnet. Das Eingabegerät 58 dient in einem sogenannten Direktsteuermodus einer quasi-direkten Steuerung/Verlagerung der Handhabungseinheit 34 und folglich des Beobachtungsinstruments 38. Mit anderen Worten kann der Bediener das Beobachtungsinstrument 38 im Direktsteuermodus (auch bezeichnet als Direct Drag Mode) durch Ziehen bzw. Schwenken/Drehen des beispielsweise griffartig, pilzartig oder knopfartig gestalteten Eingabegerätes 58 in einfacher Weise quasi-manuell steuern.

Im Direktsteuermodus wird die Handhabungseinheit 34 durch die Steuereinrichtung 44 bzw. durch deren Handhabungssteuereinheit 46 derart betrieben, dass die robotische Handhabungseinheit 34 den Bedienbefehlen unmittelbar folgt. Auf diese Weise ergibt sich für den Bediener der Eindruck, das Beobachtungsinstrument 38 direkt oder nahezu direkt im Raum zu manövrieren. Die Handhabungseinheit 34 folgt der Bewegung, also dem Steuerbefehl, des Bedieners. Sofern die Steuerbewegung durch den Bediener endet, verbleibt die Handhabungseinheit 34 in der aktuell gewählten Position und hält diese und somit auch das Beobachtungsinstrument 34 entsprechend im Raum. Im Direktsteuermodus kann die Handhabungseinheit 34 derart angesteuert werden, dass für den Bediener bei der direkten Einwirkung auf das Eingabegerät 58 eine definierte Kraft zu überwinden ist.

In einer beispielhaften Ausgestaltung ist das Eingabegerät 50 über einen Ausleger 62 mit der Plattform 22 verbunden. Der Ausleger 62 kann verschiedene Glieder aufweisen, welche verstellbar sind. Folglich kann situationsabhängig eine ergonomisch günstige Position für das Eingabegerät 50 eingestellt werden. Vorzugsweise sind die Eingabegeräte 50, 52, 54, 56, 58 signaltechnisch (also etwa über Datenleitungen oder gegebenenfalls per Funk) mittelbar oder unmittelbar mit der Steuereinrichtung 44 gekoppelt. Dies kann eine Kopplung mit der Handhabungssteuereinheit 46 und/oder der Instrumentensteuereinheit 48 umfassen.

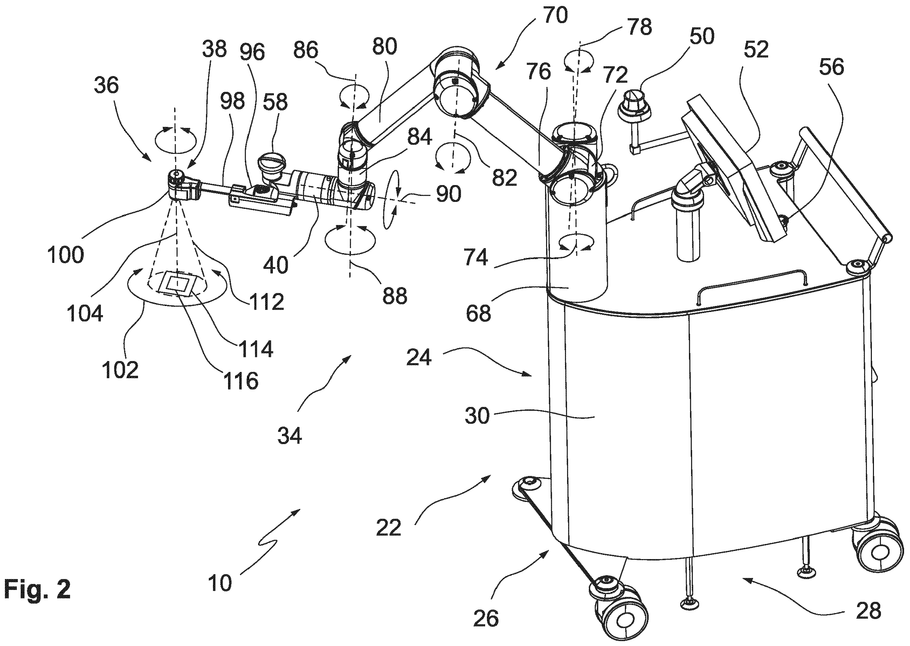

Fig. 1 und Fig. 2 veranschaulichen eine beispielhafte Ausgestaltung der robotischen Handhabungseinheit 34. Die Handhabungseinheit 34 weist ein Grundgestell 68 auf, welches an der als Wagen 34 gestalteten Plattform 22 angeordnet ist. Mit anderen Worten ist die Handhabungseinheit 34 zumindest in dem in Fig. 1 und Fig. 2 gezeigten Ausführungsbeispiel verfahrbar.

Die Handhabungseinheit 34 weist eine kinematische Kette 70 auf, deren Basis das Grundgestell 68 an der Plattform 22 bildet. Die Handhabungseinheit 34 ist als offene kinematische Kette gestaltet. Mit anderen Worten weist die kinematische Kette 70 eine Mehrzahl von Gliedern auf, welche in Reihe angeordnet und miteinander gekoppelt sind.

Die Handhabungseinheit 34 weist ein Karussell 72 auf, welches am Grundgestell 68 aufgenommen/gelagert ist. Das Karussell 72 ist beispielhaft gegenüber dem Grundgestell 68 verdrehbar (um eine vertikale Achse). Demgemäß ist zwischen dem Karussell 72 und dem Grundgestell 68 ein Gelenk 74 vorgesehen. Das Gelenk 74 definiert eine (im Ausführungsbeispiel vertikale) Rotationsachse. Das Grundgestell 68 bildet ein proximales Ende der kinematischen Kette 70 der Handhabungseinrichtung 34. Der Instrumentenhalter 40 bildet ein distales Ende der kinematischen Kette der Handhabungseinrichtung 34.

An das Karussell 72 schließt sich eine Schwinge 76 an, welche über ein Gelenk 78 mit dem Karussell 72 gekoppelt ist, vergleiche Fig. 2. Das Gelenk 78 definiert eine (im Ausführungsbeispiel horizontale) Rotationsachse. Ferner ist ein Arm 80 vorgesehen, der über ein Gelenk 82 mit der Schwinge 76 gekoppelt ist. Das Gelenk 82 definiert eine Rotationsachse. In der kinematischen Kette 70 folgt ein als Hand 84 bezeichnetes Element, welches mit dem Arm 80 über ein Gelenk 86 gekoppelt ist (vgl. Fig. 2). Das Gelenk 86 definiert eine Rotationsachse.

An das als Hand 84 bezeichnete Element schließt sich im Ausführungsbeispiel gemäß den Figuren 1 und 2 der Instrumentenhalter 40 zur Aufnahme das Beobachtungsinstrument 38 an. Der Instrumentenhalter 40 ist über ein Gelenk 88 mit dem als Hand 84 bezeichneten Element gekoppelt. Das Gelenk 88 definiert eine Schwenkachse. Der Instrumentenhalter 40 ist um die durch das Gelenk 88 definierte Achse relativ zur Hand 84 bzw. relativ zum Arm 80 rotierbar. Ferner ist es vorstellbar, dass der Instrumentenhalter 40 um seine Längsachse rotierbar ist, vergleiche das Gelenk 90, welches ebenso eine Rotationsachse definiert. Die Darstellung gemäß Fig. 2 ist nicht einschränkend zu verstehen.

Den Gelenken 74, 78, 82, 86, 88, 90 ist in einer beispielhaften Ausführungsform jeweils ein Antrieb zugeordnet. Bei dem Antrieb handelt es sich beispielsweise um einen Direktantrieb oder Servo-Antriebe. Die Antriebe sind in Fig. 2 nicht explizit dargestellt.

Es versteht sich, dass die Handhabungseinheit 34 auch abweichend von dem in den Figuren 1 und 2 gezeigten Ausführungsbeispiel gestaltet sein kann. Dies betrifft beispielsweise die Anzahl der Glieder der kinematischen Kette 70 und/oder die tatsächlichen Freiheitsgrade bzw. Bewegungsachsen zwischen benachbarten Gliedern. Wesentlich ist, dass die robotische Handhabungseinheit 34 dazu nutzbar ist, das Beobachtungsinstrument 38 in zumindest zwei Freiheitsgraden relativ zum Patienten 12 bzw. zum Tisch 14 zu bewegen. Vorzugsweise ermöglicht die Handhabungseinheit 34 eine Bewegung des Beobachtungsinstruments 38 in vier, fünf, sechs oder gar sieben Freiheitsgraden. Es versteht sich, dass auch robotische Handhabungseinheiten mit mehr oder weniger Gliedern und auch mit abweichenden Bewegungsfreiheitsgraden nutzbar sind. Die Anzahl der beweglichen (im Regelfall schwenkbaren) Achsen wird üblicherweise so gewählt, dass die gewünschten Freiheitsgrade für das Instrument 36 bereitgestellt werden können.

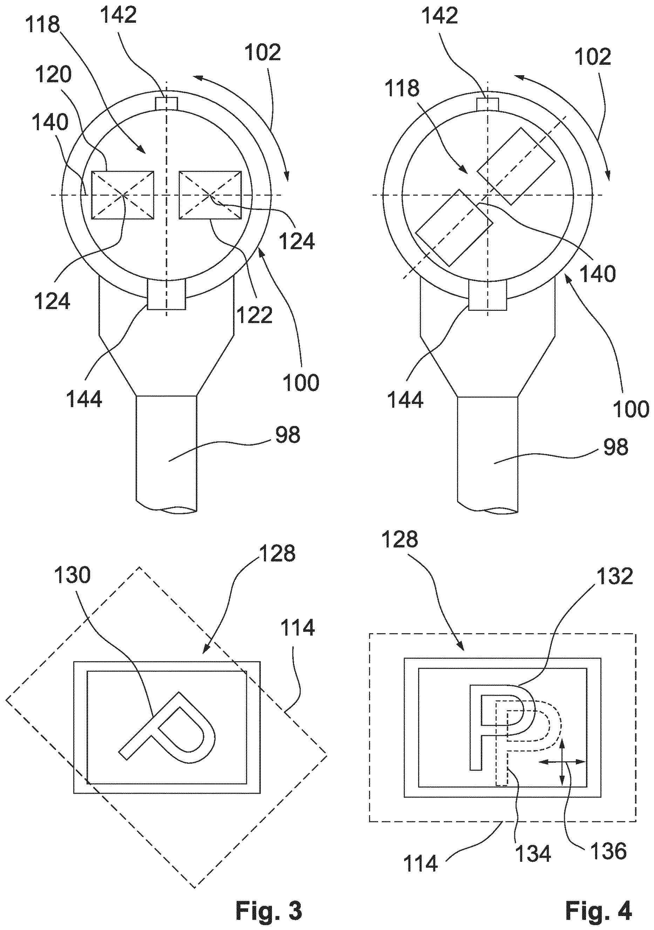

Die Figuren 3 und 4 veranschaulichen anhand vereinfachter schematischer Darstellungen eine beispielhafte Ausgestaltung eines mit 100 bezeichneten Beobachtungskopfes des Beobachtungsinstruments 38. Vergleiche hierzu auch Fig. 2. Das Beobachtungsinstrument 38 weist ein Gehäuse 96 auf. Vom Gehäuse 96 ausgehend erstreckt sich ein Schaft 98 in Richtung auf ein distales Ende des Beobachtungsinstruments 38. Am distalen Ende ist der Beobachtungskopf 100 angeordnet. Die Figuren 3 und 4 veranschaulichen lediglich das distale Ende des Beobachtungsinstruments 38 mit dem Bearbeitungskopf 100.

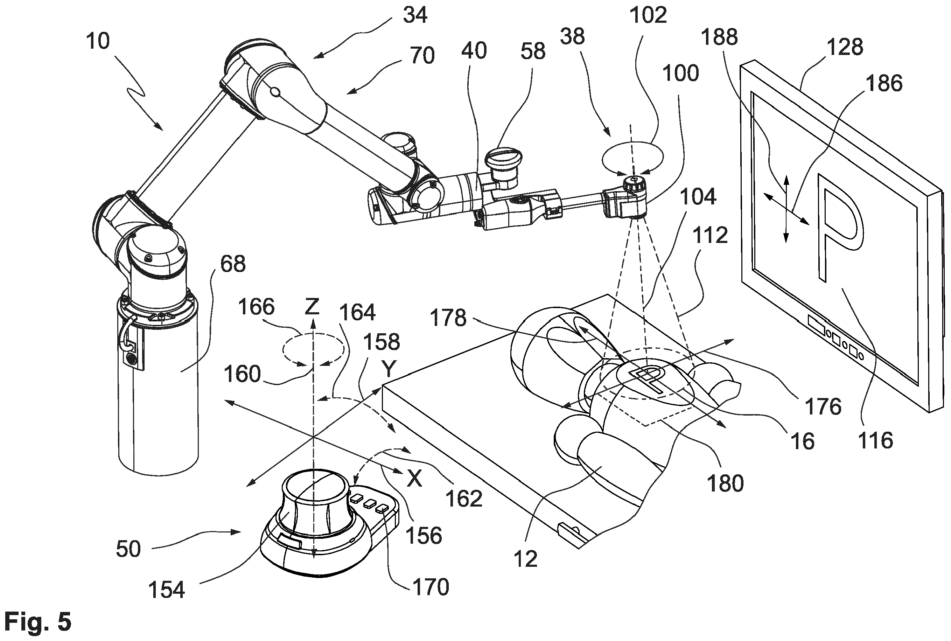

Fig. 3 und Fig. 4 ist ferner entnehmbar, dass zumindest in beispielhaften Ausgestaltungen ein Rotationsfreiheitsgrad (vergleiche den Doppelpfeil 102) für den Beobachtungskopf 100 bzw. für einen darin verbauten Bildaufnehmer vorgesehen ist. Demgemäß ist eine Bildaufrichtung (Bildrotation) in Bezug auf eine optische Achse 104 möglich, vergleiche hierzu auch Fig. 2. Das Beobachtungsinstrument 38 mit dem Beobachtungskopf 100 ist dazu ausgestaltet, ein Sichtfeld 112 (vergleiche Fig. 2 und Fig. 5) zu beobachten und im Sichtfeld 112 einen Bildausschnitt 116 in einem Aufnahmebereich 114 zu erfassen. Dies erfolgt in einer Objektebene bzw. einem Objektfeld 16 (vergleiche Fig. 1). Das Sichtfeld 112 wird durch ein optisches Abbildungssystem des Beobachtungskopfes 100 definiert.

Das Sichtfeld 112 und im Beobachtungskopf 100 verbaute Bildsensoren (ein Sensor oder mehrere Sensoren) definieren den (möglichen) Aufnahmebereich 114. Der Aufnahmebereich 114 kann nicht größer als das Sichtfeld 112 sein. Der Aufnahmebereich 114 wird etwa durch die Größe eines Bildsensors oder mehrerer Bildsensoren sowie die abbildende Optik definiert. Der Bildausschnitt 116 kann grundsätzlich dem Aufnahmebereich 114 entsprechen. Es ist jedoch auch vorstellbar, zumindest in beispielhaften Betriebsmodi, dass der Bildausschnitt 116 bewusst kleiner als der Aufnahmebereich 114 gewählt wird. Dies ist einerseits bei einem Digitalzoom vorstellbar. Ferner kann der Bildausschnitt 116 kleiner als der Aufnahmebereich 114 gewählt werden, um etwaige Abbildungsfehler/Darstellungsfehler im Randbereich des Aufnahmebereichs 114 (also an den Rändern der verbauten Bildsensoren) zu vermeiden oder zumindest zu minimieren.

Zur Erfassung des Bildausschnitts 116 bzw. des Aufnahmebereichs 114 weist das Beobachtungsinstrument 38 einen Bildaufnehmer 118 auf. In dem in den Figuren 3 und 4 veranschaulichten Ausführungsbeispiel ist ein Stereo-Bildaufnehmer 118 verbaut. Demgemäß weist der Bildaufnehmer 118 einen ersten Sensor 120 und einen zweiten Sensor 122 auf. Die Sensoren 120, 122 sind beispielsweise als CCD-Bildsensoren, CMOS-Bildsensoren oder in ähnlicher Weise gestaltet. Die Sensoren 120, 122 weisen jeweils eine Mehrzahl von Erfassungspixeln auf. Es steht sich, dass der Bildaufnehmer 118 grundsätzlich auch als (Mono-) Bildaufnehmer mit nur einem Beobachtungskanal gestaltet sein kann. Die Bezugszeichen 124 kennzeichnen jeweils ein Zentrum bzw. einen Mittelpunkt der Sensoren 120, 122.

Zur Wiedergabe des aufgenommenen Bildes ist eine Wiedergabeeinheit 128 vorgesehen. Die Wiedergabeeinheit 128 umfasst etwa einen Monitor oder ein ähnliches Display. Die Wiedergabeeinheit 128 ist in beispielhaften Ausgestaltungen zur stereoskopischen Bildwiedergabe ausgebildet. Demgemäß kann die Wiedergabeeinheit 128 als 3D-Monitor gestaltet sein. Es sind Gestaltungen vorstellbar, bei denen ein Monitor über Hilfsmittel (3D-Brillen) betrachtet wird, um den stereoskopischen Effekt zu erzielen. Es sind jedoch auch Gestaltungen vorstellbar, bei denen die Wiedergabeeinheit 128 als Head-Mounted Display (HMD), also beispielsweise als Videobrille gestaltet ist.

Ein Stereo-Bildaufnehmer 118 ermöglicht die stereoskopische Beobachtung, gegebenenfalls sogar eine 3D-Beobachtung. Dies wird durch einen Versatz zwischen den beiden Sensoren 120, 122 ermöglicht, welche an den Versatz zwischen dem rechten und dem linken Auge des Beobachters angepasst ist. Auf diese Weise ergibt sich bei der Betrachtung ein räumlicher Eindruck. Die stereoskopische Beobachtung macht es jedoch erforderlich, dass die beiden Sensoren 120, 122 in einer bestimmten Weise ausgerichtet sind, nämlich entlang eines (künstlichen) Horizonts 140, der an die Lage der Wiedergabeeinheit 128 und mittelbar an die Lage der Augen bzw. der Augenpartie des Beobachters angepasst ist.

In dem in Fig. 3 veranschaulichten Zustand zeigt die Wiedergabeeinheit 128 einen Bildausschnitt 130 in einer ersten Orientierung an. Diese ergibt sich aus der Orientierung des Bildaufnehmers 118 in Bezug auf den Beobachtungskopf 100 sowie insgesamt aus der Orientierung des Beobachtungsinstruments 38 in Bezug auf das Objektfeld 16 beim Patienten 12. Die Darstellung des Bildausschnitts 130 in Fig. 3 veranschaulicht anhand eines Beispiels (Buchstabe P) eine geneigte Orientierung, in der das unmittelbare Verständnis und vor allem die Zuordnung von Richtungen für den Beobachter erschwert ist. Es wäre für den Beobachter wünschenswert, die in Fig. 4 gezeigt Orientierung des Bildausschnitts 132 zu verwenden. Die Orientierung des Bildausschnitts 130 in Fig. 3 resultiert aus der gegebenen Orientierung des Bildaufnehmers 118, vergleiche den Horizont 140.

Um den angezeigten Bildausschnitt 132 in der gewünschten Weise auszurichten, ist es erforderlich, den Bildaufnehmer 118 mit den Sensoren 120, 122 zu drehen, vergleiche die Orientierung des Horizonts 140 des Bildaufnehmers 118 in Fig. 4. Zu diesem Zweck ist der Rotationsfreiheitsgrad 102 vorgesehen, der eine Bildaufrichtung ermöglicht. Die Bildaufrichtung unter Nutzung der Verdrehbarkeit des Bildaufnehmers 118 um die optische Achse 104 in Bezug auf den Beobachtungskopf 100 ist insbesondere für Stereo-Bildaufnehmer von Vorteil. Jedoch können sich auch bei Mono-Bildaufnehmern 118 mit nur einem Beobachtungskanal Vorteile ergeben, etwa in Bezug auf gegebene Abmessungen (zum Beispiel ein Aspektverhältnis) des verwendeten Bildsensors.

In einer beispielhaften Ausführungsform weist der Beobachtungskopf 100 einen Lagesensor/Orientierungssensor 142 zur Erfassung einer Drehlage des Bildaufnehmers 118 in Bezug auf die Beobachtungskopf 100 bzw. den Schaft 98 des Beobachtungsinstruments 38 auf. Auf dieser Basis Weise kann eine gewünschte Ausrichtung des Bildausschnitts 130, 132 in Abhängigkeit von der Ist-Orientierung des Bildaufnehmers 118 eingestellt werden.

Es ist grundsätzlich vorstellbar, den Bildaufnehmer 118 manuell um dessen optische Achse 104 zu rotieren. Es ist in alternativen Ausgestaltungen auch vorstellbar, einen Antrieb 144 zur Rotation des Bildaufnehmers 118 um die optische Achse 104 zu nutzen. Sofern ein Antrieb 144 genutzt wird, kann der Orientierungssensor 142 in den Antrieb 144 integriert werden. Es ist jedoch auch vorstellbar, die Drehlage/Orientierung des Bildaufnehmers 118 von Steuerdaten zur Steuerung des Antriebs 144 abzuleiten. Wenn also dem Antrieb 144 ein bestimmtes Drehinkrement für eine Drehbewegung vorgegeben wird, so ist im Umkehrschluss zumindest die Ziel-Orientierung des Bildaufnehmers 118 bekannt.

Es versteht sich, dass auch eine elektronische/digitale Bildaufrichtung vorstellbar ist, wobei der aufgenommene und angezeigte Bildausschnitt digital gedreht wird. Etwas Derartiges ist jedoch bei der Stereo-Beobachtung unter Beibehaltung der Stereo-Funktionalität kaum realisierbar. Jedoch ist in beispielhaften Ausführungsformen eine digitale Feinjustierung oder Feinausrichtung vorstellbar.

Es versteht sich, dass das Beobachtungsinstrument 38 grundsätzlich auch über die robotische Handhabungseinheit 34 ausgerichtet werden könnte, um den angezeigten Bildausschnitt 130, 132 auszurichten. Dies würde jedoch häufig dazu führen, dass das Beobachtungsinstrument 38 bzw. die Handhabungseinheit 34 im Wege stehen und etwa die freie direkte Sicht auf das Operationsfeld/Objektfeld für den Operateur und/oder Dritte beeinträchtigen könnten. Ferner muss das Operationsfeld in vielen Fällen für weitere Instrumente zugänglich sein. Deshalb wird die robotische Handhabungseinheit 34 regelmäßig derart ausgerichtet, dass diese so wenig wie möglich den Arbeitsablauf stört. Dann muss jedoch unter Umständen der Bildaufnehmer 118 unter Nutzung des Freiheitsgrades 102 rotiert werden, um das Bild in der gewünschten Weise aufzurichten.

Diese Ausrichtung/Aufrichtung durch Rotation des Bildaufnehmers 118 führt jedoch unter Umständen dazu, dass die robotische Handhabungseinheit 34 nicht intuitiv gesteuert werden kann. Wenn nämlich der Beobachter/Bediener beispielsweise über das Eingabegerät 50 Steuerbefehle in Form von Richtungsbefehlen und Wegbefehlen vorgibt, um über die Handhabungseinheit 34 das Beobachtungsinstrument 38 zu verfahren, so orientiert er sich regelmäßig am angezeigten Bildausschnitt 116 (vergleiche auch Bezugszeichen 130, 132 in den Figuren 3 und 4). Die Ausrichtung des Motivs im Bildausschnitt 116 korreliert jedoch häufig nicht mit den Betätigungsachsen (zum Beispiel rechts - links - vorne - hinten) des Eingabegerätes 50. In einem solchen Fall führt also eine Rechtsbewegung am Eingabegerät 50 nicht unbedingt zu einer korrespondierenden Bewegung des angezeigten Bildausschnitts 116 nach rechts.

Fig. 3 und Fig. 4 veranschaulichen ferner eine beispielhafte Ausgestaltung, bei der das angezeigte Bild bzw. der angezeigte Bildausschnitt 116, 132 kleiner als der theoretisch verfügbare Aufnahmebereich 114 der Sensoren 120, 122 ist. Wenn also nur ein Ausschnitt des jeweiligen Aufnahmebereichs 114 angezeigt wird, so ist es theoretisch möglich, den angezeigten Bildausschnitt 116 innerhalb des Aufnahmebereichs 114 zu verschieben. Dies wird in Fig. 4 durch einen verschobenen Bildausschnitt 134 sowie ein mit 136 bezeichnetes Koordinatensystem angedeutet. Ferner ermöglicht dies, wie zuvor schon angedeutet, zumindest in Grenzen einen sogenannten Digital-Zoom.

Die Orientierung des Aufnahmebereichs 114 in Fig. 3 (in Relation zum durch die Wiedergabeeinheit 128 angezeigten Bildausschnitt 116, 130) verdeutlicht, dass auch bei einer digitalen Bildrotation ein größerer Aufnahmebereich 114 von Vorteil ist. Somit kann der angezeigte Bildausschnitt 116, 130 bei dem gegebenen Aspektverhältnis bzw. allgemein bei der gegebenen Gestalt der Wiedergabeeinheit 128 gedreht werden, ohne dass es beispielsweise in den Ecken der Wiedergabeeinheit 128 zu Auslassungen kommt.

Die Möglichkeit, den Bildausschnitt 116, 132 kleiner als den Aufnahmebereich 114 zu wählen, führt zu Situationen, in denen ein aktuelles Zentrum bzw. ein aktueller Mittelpunkt des angezeigten Bildausschnitts 116, 132 nicht dem Mittelpunkt 124 des Sensors 120, 122 entspricht. Dies muss bei dem Betrieb der Handhabungsvorrichtung 10 berücksichtigt werden.

Gemäß einem Aspekt der vorliegenden Offenbarung wird vorgeschlagen, eine Koordinatentransformation zwischenzuschalten, um eine intuitive Steuerung der Handhabungseinheit 34 zum Verfahren des Instruments 36 bzw. Beobachtungsinstruments 38 zu ermöglichen. Dieser Ansatz erlaubt es beispielsweise, sich bei der Steuerung am angezeigten Bildausschnitt 116 zu orientieren, insbesondere an dessen Orientierung.

Dies wird mit ergänzendem Bezug auf Fig. 5 veranschaulicht. Fig. 5 zeigt einen Zustand, in dem der Bildaufnehmer (nicht explizit gezeigt) im Beobachtungskopf 100 des Beobachtungsinstruments 38 derart um die optische Achse 104 ausgerichtet ist, dass der angezeigte Bildausschnitt 116 bei der Wiedergabeeinheit 128 in der gewünschten Orientierung angezeigt ist. Diese Orientierung wird nun der Steuerung der robotischen Handhabungseinheit 34 zugrunde gelegt.

Die Steuerung der Handhabungseinheit 34 erfolgt beispielsweise über ein Betätigungselement 154 des Eingabegerätes 50. Beispielhaft ist das Betätigungselement 154 knopfartig, tellerartig oder puckartig gestaltet. Das Betätigungselement 154 kann jedoch auch ähnlich einem Joystick gestaltet sein. Alternativ kann das Betätigungselement 154 ähnlich einem sogenannten Drehdrücksteller gestaltet sein. Das Betätigungselement 154 weist verschiedene Bewegungsachsen bzw. Eingabeachsen auf. Über diese Eingabeachsen können Steuerbefehle erzeugt werden, indem der Bediener in der gewünschten Weise auf das Betätigungselement 154 einwirkt. Vorzugsweise ist das Betätigungselement 154 als Mehrachs-Betätigungselement gestaltet. Demgemäß ist das Betätigungselement 154 beispielsweise dazu ausgestaltet, Bewegungen entlang mehrerer linearer Achsen 156, 158, 160 zu erfassen. Insgesamt sind beispielsweise sechs Freiheitsgrade denkbar, beispielsweise drei translatorische und drei rotatorische Freiheitsgrade.

Beispielhaft kann die Achse 156 als Translationsachse bezeichnet werden. Die Achse 156 ist beispielhaft einer X-Richtung zugeordnet. Entlang der Achse 156 kann eine Schubbewegung/Linearbewegung induziert werden. Es versteht sich, dass das Betätigungselement 154 entlang der Achse 156 nur in geringem Maße auslenkbar ist. Beispielhaft kann die Achse 158 als Translationsachse bezeichnet werden. Die Achse 158 ist beispielhaft einer Y-Richtung zugeordnet. Entlang der Achse 158 kann eine Schubbewegung/Linearbewegung induziert werden. Es versteht sich, dass das Betätigungselement 154 entlang der Achse 158 nur in geringem Maße auslenkbar ist. Beispielhaft kann die Achse 160 als Hubachse bezeichnet werden. Die Achse 160 ist beispielhaft einer Z-Richtung zugeordnet. Entlang der Achse 160 kann eine Schubbewegung/Linearbewegung induziert werden. Es versteht sich, dass das Betätigungselement 154 entlang der Achse 160 nur in geringem Maße auslenkbar ist.

Dies heißt mit anderen Worten, translatorische Bewegungen des Beobachtungskopfes 100 des Beobachtungsinstruments 38 in einer Ebene (etwa einer X-Y-Ebene) können durch leichte Bewegungen des Betätigungselements 154 entlang der Achsen 156, 158 bewirkt werden.

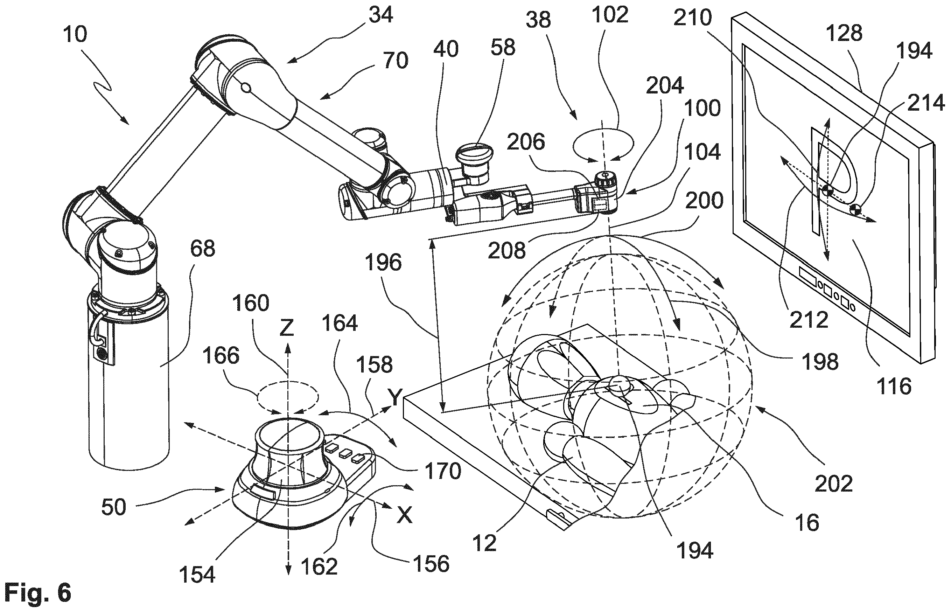

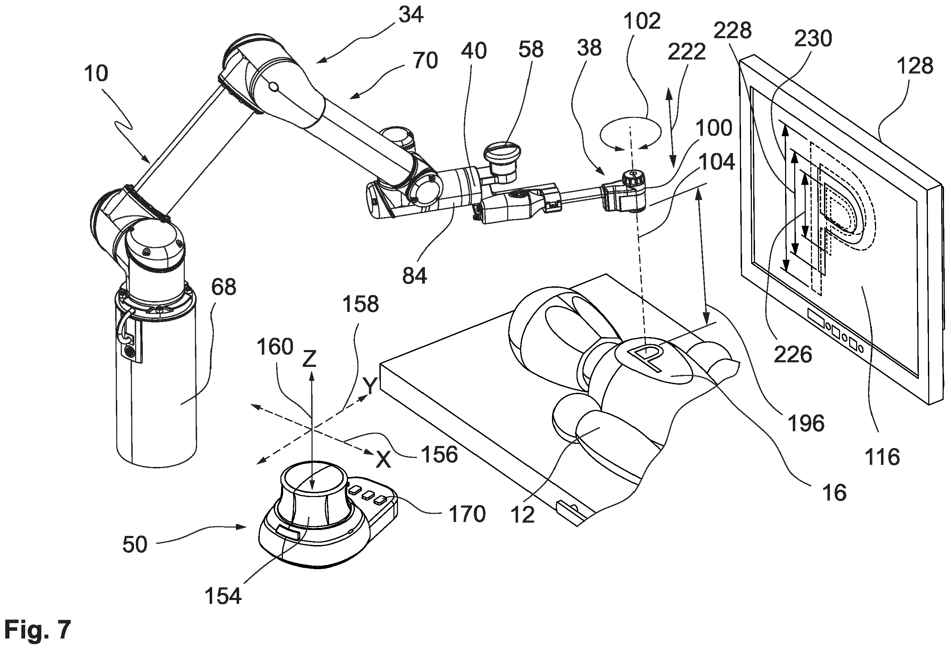

Die Hubachse 160 kann beispielsweise dazu genutzt werden, einen Objektabstand (Bezugszeichen 196 in Fig. 6 und Fig. 7) zwischen dem Beobachtungsinstrument 38 und dem Objektfeld 16 zu verändern. Grundsätzlich ist auch eine Nutzung einer Bewegung entlang der Hubachse 160 (Z-Richtung) zur Steuerung eines Fokusantriebs vorstellbar.

Daneben weist das Betätigungselement 154 gemäß dem in Fig. 5 veranschaulichten Ausführungsbeispiel Schwenkachsen bzw. Rotationsachsen 162, 164, 166 auf. Die Schwenkachse 162 beschreibt Schwenkbewegungen um die Achse 156, also beispielsweise um die X-Achse. Die Schwenkachse 164 beschreibt Schwenkbewegungen um die Achse 158, also beispielsweise um die Y-Achse. Die Schwenkachse oder Rotationsachse 166 beschreibt Schwenkbewegungen/Rotationsbewegungen um die Achse 160, also um die Z-Achse.

Die Schwenkachsen 162, 164 können beispielsweise dazu genutzt werden, dass an der robotischen Handhabungseinheit 34 aufgenommene Beobachtungsinstrument 38 in Bezug auf das Objektfeld 16 zu kippen. Dies erfolgt über eine entsprechende Ansteuerung der Handhabungseinheit 34 in Reaktion auf Schwenkbewegungen um die Schwenkachsen 162, 164, die der Bediener am Betätigungselement 154 vornimmt. In einer beispielhaften Ausgestaltung wird das Beobachtungsinstrument 38 um den Fokuspunkt, also den eingestellten Arbeitsabstand geschwenkt (Pivotbewegung).

Die Rotationsachse 166 kann beispielsweise dazu genutzt werden, einen Fokusantrieb des Beobachtungsinstruments 38 zu steuern. Grundsätzlich ist auch vorstellbar, einen Arbeitsabstand/Objektabstand (Bezugszeichen 196 in Fig. 6 und Fig. 7) zwischen dem Beobachtungsinstrument 38 und dem Objektfeld 16 in Reaktion auf Bedienereingaben (Drehbewegungen) zu verändern. Zwischen diesen Betriebsarten kann unter Nutzung eines Freigabeschalters umgeschaltet werden. Beispielhaft wird die Achse 160 für eine Veränderung des Arbeitsabstands und die Achse 166 für die Steuerung des Fokusantriebs genutzt. Umgekehrt ist es vorstellbar, die Achse 160 für die Steuerung des Fokusantriebs und die Achse 166 für die Veränderung des Arbeitsabstands zu nutzen.

Grundsätzlich ist es vorstellbar, das Betätigungselement 154 in mehreren Raumrichtungen auslenkbar zu gestalten. Auf diese Weise ergibt sich eine eindeutige Bedienung für den Bediener. Es ist jedoch auch vorstellbar, eine Krafteinwirkung auf das Betätigungselement 154 zu erfassen, etwa über geeignete Kraftsensoren. In einem solchen Fall ist das Betätigungselement 154 nicht unbedingt makroskopisch auslenkbar. Stattdessen liegt er eine mikroskopische Auslenkbarkeit vor. Auch auf diese Weise können Bewegungen erfasst sowie den Achsen 156, 158, 160, 162, 164, 166 zugeordnet und auf dieser Basis in Steuerbefehle überführt werden.

Das Eingabegerät 50 weist beispielhaft weitere Betätigungselemente 170 in Form von Tasten oder Knöpfen auf. Auf diese Weise können weitere Funktionen gesteuert werden. Insbesondere können bestimmte Befehle quittiert werden. Ferner ist eine Auswahl eines aktuellen Operationsmodus des Eingabegerätes 50 über eines der Betätigungselemente 170 vorstellbar. Eine weitere Verwendungsmöglichkeit für die Betätigungselemente ist die Speicherung aktueller Positionen der Handhabungseinheit 34 bzw. des Beobachtungsinstruments 38, wobei bedarfsweise die gespeicherte Position ausgehend von einer zwischenzeitlich eingenommenen Position angefahren wird. Sowohl das Speichern einer Position als auch das Anfahren einer zuvor gespeicherten Position kann durch die Betätigungselemente 170 bewirkt werden. Das Anfahren der zuvor gespeicherten Position kann sich einerseits auf die Zielposition beschränken. Alternativ kann das Anfahren der zuvor gespeicherten Position derart erfolgen, dass der zuvor genutzte Bewegungspfad "rückwärts" abgefahren wird.

Im Einklang mit der in Fig. 5 veranschaulichten Beispielkonfiguration erfolgt zur Vereinfachung der Steuerung eine Koordinatentransformation bzw. ein Abgleich der Orientierungen/Koordinatensysteme.

In Fig. 5 veranschaulichen in das Objektfeld 16 projizierte Doppelpfeile 176, 178 ein Koordinatensystem, dass die Orientierung des Bildaufnehmers widerspiegelt. Demgemäß ist es in dem in Fig. 5 gezeigten Betriebsmodus gewünscht, das Beobachtungsinstrument 38 und folglich das Objektfeld 16 in einer Ebene 180 in Reaktion auf Bedienbefehle am Eingabegerät 50 zu verfahren. Eine solche Bewegung in der Ebene 180 erfolgt durch Interpolation und entsprechende Ansteuerung der Glieder der kinematischen Kette 70 der robotischen Handhabungseinheit 34.

In dem an der Wiedergabeeinheit 128 angezeigten Bildausschnitt 116 sind resultierende Bewegungsachsen 186, 188 angedeutet. In dem veranschaulichten beispielhaften Betriebsmodus ist die Achse 156 am Eingabegerät 50 der resultierenden Achse 186 zugeordnet. Ferner ist die Achse 158 am Eingabegerät 50 der resultierenden Achse 188 im Bildausschnitt 116 zugeordnet. Demgemäß wird die robotische Handhabungseinheit 34 derart angesteuert, dass bei einer Rechts-Links-Bewegung am Eingabeelement 154 der angezeigte Bildausschnitt 116 ebenso nach rechts oder nach links verfahren wird. Ferner erfolgt die Ansteuerung der robotischen Handhabungseinheit 34 derart, dass bei einer Vor-Zurück-Bewegung am Eingabeelement 154 der angezeigte Bildausschnitt 116 entlang der angedeuteten Achse 188 hoch oder runter verfahren wird. Eine derartige Bedienung ist intuitiv und kann unter Beobachtung des angezeigten Bildausschnitts 116 an der Wiedergabeeinheit 128 erfolgen.

Dieser Betriebsmodus erfordert jedoch eine Erfassung einer aktuellen Orientierung (gekrümmter Doppelpfeil 102) des Bildaufnehmers im Beobachtungskopf 100 und eine Berücksichtigung dieser Ausrichtung (vergleiche die Doppelpfeile 176, 178) bei der Ansteuerung der robotischen Handhabungseinheit 34. Dies gilt insbesondere bei Verwendung eines Stereo-Bildaufnehmers 118 (vergleiche Fig. 2 und Fig. 3). In Fig. 5 ist beispielsweise der Horizont 140 des Bildaufnehmers 118 (vergleiche erneut Fig. 2 und Fig. 3, vergleiche ebenso den Doppelpfeil 176 in Fig. 5) parallel zur interpolierten Achse 176 ausgerichtet. Diese Ausrichtung wird bei der Interpolation der Bewegungsbahnen für die gewünschten X-Y-Bewegungen in der Ebene 180 berücksichtigt. Mit anderen Worten erfolgt die Koordinatentransformation bei der Umsetzung von Bedienbefehle am Eingabegerät 50 vorzugsweise derart, dass die Achsen 156, 176, 186 hinsichtlich der Steuerbefehle an die Handhabungseinheit 34 parallel zueinander ausgerichtet sind, und dass die Achsen 158, 178, 188 hinsichtlich der Steuerbefehle an die Handhabungseinheit 34 parallel zueinander ausgerichtet sind. Sodann kann in einfacher Weise ein Verschieben des angezeigten Bildausschnitts 116 in der Ebene 180 erfolgen.

Der Bediener kann sich also unabhängig von der äußerlichen Orientierung/Lage der Handhabungseinheit 34 bzw. des Beobachtungsinstruments 38 an der Ausrichtung des Bildausschnitts 116 an der Anzeige der Wiedergabeeinheit 128 orientieren, um den Bildausschnitt 116 über Bedienereingaben an dem zugeordneten Eingabeachsen 156, 158 des Eingabegerätes 50 intuitiv in zumindest zwei Achsen zu steuern.

Zumindest in beispielhaften Ausführungsformen werden während dieses speziellen Verfahrmodus andere Freiheitsgrade des Betätigungselements 154 des Eingabegerätes 50 nicht berücksichtigt, so dass eine ideale oder nahezu-ideale Bewegung in der Ebene 180 ermöglicht ist.

Es versteht sich, dass auch andere Betriebsmodi vorstellbar sind, etwa auch ein räumlicher Modus oder 3D-Modus in dem über das Eingabegerät 50 eine räumliche Steuerung/Bewegung des Beobachtungsinstruments 38 in drei oder mehr Raumachsen (Translationsachsen/Linearachsen und Schwenkachsen/Rotationsachsen) möglich ist.

Fig. 5 veranschaulicht einen Modus, bei dem das Beobachtungsinstrument 38 möglichst parallel mit konstantem Abstand zum beobachteten Objektfeld 16 bewegt wird. Der Objektabstand (vergleiche erneut das Bezugszeichen 196 in Fig. 6 und Fig. 7) bleibt im Wesentlichen gleich.

In Ergänzung hierzu veranschaulicht Fig. 6 einen weiteren Modus, in dem das Beobachtungsinstrument 38 entlang eines gekrümmten Pfades bzw. einer gekrümmten Fläche/Schale in Bezug auf einen Pivotpunkt 194 im Objektfeld 16 bewegt wird. Beispielhaft handelt es sich bei dem Pivotpunkt 194 um ein Zentrum des - einen Teil des Objektfeldes 16 wiedergegebenen - Bildausschnitts 116. Die Bewegung entlang der gekrümmten Schale (beispielsweise Kugelschale oder Kugelhalbschale) erfolgt zumindest in beispielhaften Ausgestaltungen unter Berücksichtigung eines konstanten Objektabstands 196.

Mit anderen Worten kann die Bewegung des Beobachtungsinstruments 38 entlang inter-polierter gekrümmter Achsen 198, 200 erfolgen, die einer sphärischen Fläche 202 (Kugel oder Kugelabschnitt) zugeordnet sind. Beispielhaft, ohne dass dies einschränkend zu verstehen ist, ist die Achse 198 einem 0°-Längengrad und die Achse 200 einem 90°-Längengrad zugeordnet. Die Bewegung entlang/auf der sphärischen Fläche 202 erfolgt unter Wahrung einer Ausrichtung mit der optischen Achse 104 auf den gewählten Pivotpunkt 194. Der Pivotpunkt 194 kann sich aus einem aktuellen Zentrum des beobachteten Bildausschnitts 116 ergeben. Der Pivotpunkt 194 kann jedoch auch außermittig im Bildausschnitt 116 angeordnet und gewählt werden. Der Pivotpunkt 194 kann grundsätzlich auch als Fokuspunkt bezeichnet werden.

In einer beispielhaften Ausführungsform weist das Beobachtungsinstrument 38 beim Beobachtungskopf 100 eine Beobachtungsoptik 204 auf. Beispielhaft ist der Beobachtungsoptik 204 ein Fokusantrieb 206 zur Fokusverstellung zugeordnet. Der Fokusantrieb 206, dient dazu, einen Fokusabstand der Beobachtungsoptik 204 an den gewählten Arbeitsabstand/Objektabstand 196 anzupassen, so dass das aktuell beobachtete Objektfeld 16 hinreichend scharf abgebildet wird. Die Steuerung des Fokusantriebs 206 kann manuell und/oder automatisch erfolgen.

Das Beobachtungsinstrument 38 weist zumindest in beispielhaften Ausführungsformen ferner eine Messeinrichtung 208 zur Ermittlung des Objektabstands 196 auf. Auf diese Weise kann der aktuelle Objektabstand 196 bestimmt werden. Bei der in Fig. 6 veranschaulichten Bewegung des Beobachtungsinstruments 38 entlang der gekrümmten Bahn bzw. Fläche 202 soll der Objektabstand 196 konstant gehalten werden. Dafür muss jedoch zumindest in einigen Ausführungsbeispielen der Objektabstand 196 zunächst noch ermittelt werden. Dies erfolgt über die Messeinrichtung 208.

In einer alternativen Ausgestaltung kann die Steuereinrichtung 44 den Objektabstand 196 mittelbar über aktuelle Betriebsparameter der Beobachtungsoptik 204 bzw. des Fokusantriebs 206 ermitteln. Mit anderen Worten lässt also ein bestimmter Zustand der Beobachtungsoptik 204 auf einen bestimmten Objektabstand 196 schließen.

Beispielhaft erfolgt die Steuerung der Fahrbewegung entlang der gekrümmten Achsen 198, 200 unter Nutzung der Schwenkachsen 162, 164 des Eingabegerätes 50. Mit anderen Worten kann etwa ein Verschwenken des Betätigungselements 154 um die X-Achse 156 (Schwenkachse 162) eine Verfahrbewegung entlang der gekrümmten Achse 198 steuern. Demgemäß kann etwa ein Verschwenken des Betätigungselements 154 um die Y-Achse 158 (Schwenkachse 164) eine Verfahrbewegung entlang der gekrümmten Achse 200 steuern. In Fig. 6 sind resultierende Bewegungen des angezeigten Bildausschnitts 116 an der Wiedergabeeinheit 128 durch gekrümmte Doppelpfeile 210, 212 angedeutet. Es versteht sich, dass die Wiedergabeeinheit 128 im Normalfall einen ebenen Bildschirm aufweist. Insofern sind die Pfeile 210, 212 lediglich aus Veranschaulichungsgründen gekrümmt.

Mit anderen Worten erlaubt der in Fig. 6 gezeigte Modus der Handhabungsvorrichtung 10 ein Umkreisen des Zentrums des Bildausschnitts 116 mit dem Beobachtungsinstrument 38, wobei das Beobachtungsinstrument 38 mit seiner optischen Achse 104 auf das Zentrum ausgerichtet bleibt. Ähnlich einem Planeten kann das Beobachtungsinstrument 38 das Zentrum umkreisen, wobei die optische Achse 104 beispielsweise radial auf das Zentrum ausgerichtet bleibt.

Erneut gilt, dass gerade bei einem Stereo-Bildaufnehmer 118 (Fig. 3 und Fig. 4) die aktuelle Ausrichtung des Bildaufnehmers 118 (Horizont 140) bestimmt und etwa mittels Koordinatentransformation bei der Steuerung berücksichtigt wird, so dass sich der Bediener intuitiv am angezeigten Bildausschnitt 116 orientieren kann. Unter Nutzung der Schwenkachsen 162, 164 erfasste Steuerimpulse führen zu richtungsgleichen Bewegungen des angezeigten Bildausschnitts 116 entlang der resultierenden Achsen/Pfade 210, 212. Die Bedienung wird deutlich vereinfacht.

Es ist grundsätzlich vorstellbar, den Pivotpunkt 194 auch im Zentrum des angezeigten Bildausschnitts 116 zu verorten. Dies kann damit einhergehen, dass der Pivotpunkt 194 schlussendlich auch mit dem Zentrum 124 des Sensors 120, 122 des Bildaufnehmers 118 (vergleiche Fig. 3) zusammenfällt. Diese Weise hat man eine doppelt zentrale Ausrichtung.

Wie vorstehend bereits in Zusammenhang mit Fig. 4 erläutert, kann es auch Situationen geben, in denen das Zentrum des angezeigten Bildausschnitts 116 nicht mit dem Zentrum 124 des Sensors 120, 122 übereinstimmt. In einem solchen Fall ist es gleichwohl vorstellbar, dass der Pivotpunkt 194 im Zentrum des angezeigten Bildausschnitts 116 gewählt wird. Demgemäß liegt ein Versatz zwischen dem Pivotpunkt 194 und dem tatsächlichen Zentrum 124 des Sensors 120, 122 vor. Dieser Versatz wird von der Steuereinrichtung 44, insbesondere von der Handhabungssteuereinrichtung 46, bei der Bewegungsteuerung des Beobachtungsinstruments 38 entlang der gekrümmten Bahn 198, 200 berücksichtigt. Gleichwohl ist das Ziel der Steuerung, den gewählten Pivotpunkt 194 im Zentrum des angezeigten Bildausschnitts 116 zu halten.

Es ist jedoch auch vorstellbar, dass ein Pivotpunkt 214 gewählt wird, der bewusst nicht im Zentrum des angezeigten Bildausschnitts 116 liegt. Es gibt also zwischen dem Zentrum des angezeigten Bildausschnitts 116 und dem gewählten außermittigen Pivotpunkt 214 einen Versatz. Gedanklich wird also ein außermittiger Ankerpunkt als Zentrum der Bewegung des Beobachtungsinstruments auf der gekrümmten Fläche 202 gewählt. Die Steuereinrichtung 44, insbesondere die Handhabungssteuereinrichtung 46, kann dazu ausgestaltet sein, diesen an der Anzeige der Wiedergabeeinheit 128 sichtbaren Versatz während der Bewegung beizubehalten. Mit anderen Worten ist die optische Achse 104 des Beobachtungsinstruments 38 in diesem Modus bewusst nicht auf das Drehzentrum, also den außermittigen Pivotpunkt 214 ausgerichtet.

Es versteht sich, dass die in Fig. 5 und Fig. 6 gezeigten Bedienmodi kombiniert werden können, wenn der Bediener das Betätigungselement 154 sowohl translatorisch (Achsen 156, 158) als auch rotatorisch (Achsen 162, 164) bewegt. Es ist jedoch auch vorstellbar, die beiden Bedienmodi voneinander zu trennen. Demgemäß gibt es einen Parallelverschiebemodus, einen Schwenkmodus, sowie einen kombinierten Modus. Die entsprechenden Modi können beispielhaft über die Betätigungselemente 170 am Eingabegerät 50 oder anderweitig ausgewählt werden.

Fig. 7 veranschaulicht die Nutzung des Eingabegerätes 50 zur Steuerung weiterer Funktionen des optischen Beobachtungsinstruments 38. Beispielhaft kann die Hubachse 160 des Eingabegerätes 50 genutzt werden, um eine Veränderung des Objektabstands 196 herbeizuführen, vergleiche die interpolierte Achse 222. Über diese Funktion kann eine Vergrößerung (Detailerhöhung) bzw. Erhöhung des Abbildungsmaßstabs bewirkt werden, wenn der Beobachtungskopf 100 mit dem Bildaufnehmer 118 näher an die Objektebene/das Objektfeld 16 heranfährt.

Das Resultat einer Veränderung des Arbeitsabstands/Objektabstands 196 wird durch die Doppelpfeile 226, 228, 230 im angezeigten Bildausschnitt 116 veranschaulicht. Wenn der Arbeitsabstand verringert wird, erscheint das wiedergegebene Bild größer. Wenn der Arbeitsabstand erhöht wird, erscheint das wiedergegebene Bild kleiner. Auf diese Weise kann -jedenfalls vom Resultat her betrachtet - eine Zoom-Funktion bewirkt werden. Dies kann über Manipulationen am Betätigungselement 154 bewirkt werden. Dies kann einerseits ein Drücken oder Ziehen entlang der Hubachse 160 (Z-Achse) umfassen. Es ist jedoch auch vorstellbar, diese Funktion über ein Verdrehen des Betätigungselements 154 um die Z-Achse 160 (Rotationsachse 166) herbeizuführen.

Es ist zumindest in beispielhaften Ausführungsformen erforderlich, auch die Gegenstandsweite der optischen Einheit des Beobachtungskopfes 100 anzupassen, damit das Bild bei dem gewählten Objektabstand auch scharf erscheint. Auch hierfür kann einer der Bewegungsfreiheitsgrade (vergleiche die Achsen 160, 166) des Betätigungselements 154 des Eingabegerätes 50 zur Steuerung eines Fokusantriebs genutzt werden.

In alternativen Ausführungsformen ist bei dem Beobachtungskopf 100 eine optische Einheit mit variabler Brennweite verbaut, so dass ein optischer Zoom realisierbar ist. In alternativen Ausführungsformen ist zumindest in Grenzen ein sogenannter Digital-Zoom möglich. Dies ist insbesondere dann der Fall, wenn der wiedergegebene Bildausschnitt 116 kleiner als der theoretisch mögliche Aufnahmebereich 114 des Bildaufnehmers bzw. kleiner als das Sichtfeld der optischen Baugruppe ist. Dann kann der erfasste und/oder wiedergegebene Bildausschnitt 116 innerhalb der durch den Aufnahmebereich 114 vorgegebenen Grenzen zumindest ein Stück weit variiert werden, um eine vergrößerte Detaildarstellung oder verkleinerte Übersichtsdarstellung zu ermöglichen.

Ferner ist es vorstellbar, den Digital-Zoom mit alternativen Maßnahmen zur Bereitstellung vergrößerter/verkleinerter Bildausschnitte zu koppeln, um über ein und dasselbe Eingabegerät 50 eine intuitive Steuerung einer solchen Vergrößerungsfunktion zu ermöglichen. Der Modus, in dem das Eingabegerät 50 genutzt wird, um durch eine Bewegung des Beobachtungsinstruments 38 entlang der interpolierten Achse 222 zur Veränderung des Objektabstands 196 herbeizuführen, kann grundsätzlich gleichzeitig mit den in Fig. 5 und Fig. 6 beschriebenen Modi verwendet werden. Es ist jedoch auch vorstellbar, die einzelnen Modi voneinander zu trennen, um eine eindeutige Bedienung zu ermöglichen.

Ferner ist es in einer beispielhaften Ausgestaltung vorstellbar, eine Verfahrgeschwindigkeit der robotischen Handhabungseinheit und folglich des daran aufgenommenen Beobachtungsinstruments 38 in Abhängigkeit von einer gewählten Zoom-Stufe bzw. in Abhängigkeit von einem gewählten Arbeitsabstands/Objektabstand 196 zu wählen. Demgemäß kann das Beobachtungsinstrument 38 langsamer verfahren werden, wenn eine vergrößerte Darstellung (hoher Zoom-Faktor, geringer Objektabstand bzw. großer Abbildungsmaßstab) gewählt ist. Auf diese Weise kann der sich ändernde Bildausschnitt 116 noch gut für den Beobachter erfasst werden. Umgekehrt kann das Beobachtungsinstrument 38 schneller verfahren werden, wenn eine verkleinerte Darstellung (kleiner Zoom-Faktor, großer Objektabstand bzw. kleiner Abbildungsmaßstab) gewählt ist. Dies ist möglich, da der angezeigte Bildausschnitt 116 einen größeren Bereich des Objektfeldes 16 abdeckt.

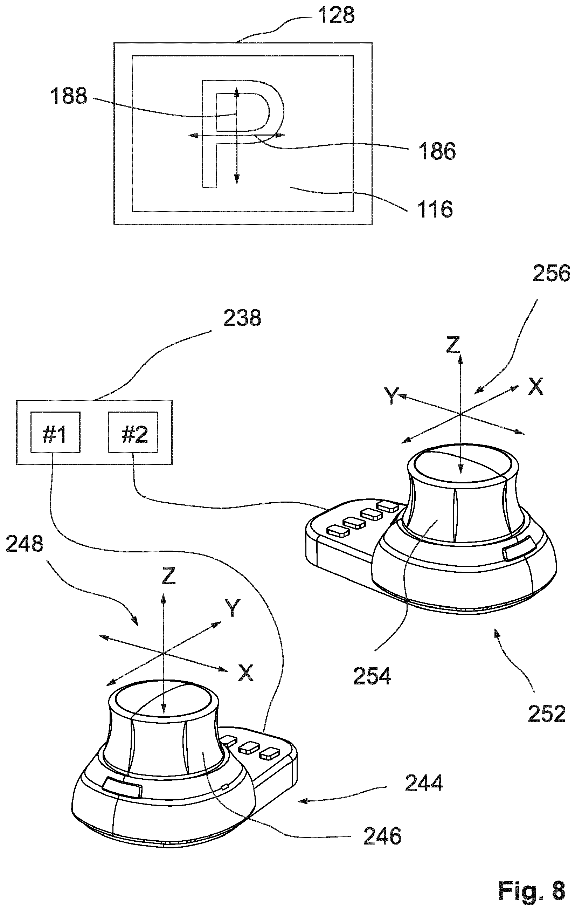

Fig. 8 veranschaulicht in Zusammenschau mit Fig. 1, dass eine Mehrzahl von Eingabegeräten 244, 252 über eine Schnittstelle 238 mit der Steuereinrichtung 44, also etwa der Handhabungssteuereinheit 46 oder der Instrumentensteuereinheit 48 gekoppelt werden kann. In Fig. 8 handelt es sich bei den Eingabegeräten 244, 252 jeweils um Einhand-Eingabegeräte mit einem Eingabeelement 246, 254, welches in diversen Raumachsen betätigbar ist, um Steuerbefehle zu erzeugen. Vergleiche hierzu die den Eingabeelementen 246, 254 zugeordneten Koordinatensysteme 248, 256. Eine Mehrzahl von Eingabegeräten 244, 252 kann erforderlich sein, um verschiedenen Bedienern in der Operationsumgebung Manipulationen zu ermöglichen. Dies kann einerseits eine Steuerung der robotischen Handhabungseinheit 34 betreffen. Ferner kann dies eine Steuerung des Beobachtungsinstruments 38 betreffen, etwa zur Steuerung von Bildaufnahmeparametern und/oder Bildwiedergabeparametern.

Es versteht sich, dass die Steuereinrichtung 44 mit der Schnittstelle 238 eine geeignete Aktivierung/Priorisierung/Hierarchie nutzt, um in eindeutiger Weise zu definieren, welches der Eingabegeräte 244, 252 aktuell verwendet wird. Auf diese Weise kann beispielsweise ein primäres Eingabegerät 244 und ein sekundäres Eingabegerät 252 definiert werden, wobei das primäre Eingabegerät 244 höher priorisiert ist. Demgemäß wird das sekundäre Eingabegerät 252 deaktiviert bzw. werden dessen Befehle ignoriert, wenn das primäre Eingabegerät 244 genutzt wird. Andere Maßnahmen zur Definition des aktuell genutzten Eingabegerätes sind vorstellbar.

Die medizinische Handhabungsvorrichtung 10 kann grundsätzlich über verschiedenartige Eingabegeräte angesteuert werden, vergleiche die Eingabegeräte 50, 52, 54, 56 und 58 in Fig. 1. Fig. 8 zeigt zwei Eingabegeräte 244, 252 des gleichen Typs. Dies ist jedoch nicht einschränkend zu verstehen.

Unabhängig von der aktuellen Position und Orientierung der Eingabegeräte 244, 252 können sich deren Benutzer bei der Steuerung der robotischen Handhabungseinheit 34 am aktuellen Bildausschnitt 116 der Wiedergabeeinheit 128 orientieren. Mit anderen Worten werden die Koordinatensysteme 248, 256 der Eingabegeräte 244, 252 mit den resultierenden Bewegungsachsen 186, 188 des angezeigten Bildausschnitts 116 in Übereinstimmung gebracht.

Ferner ist es vorstellbar, unterschiedliche Wiedergabeeinheiten 128, etwa verschiedene Monitore oder HMDs für die verschiedenen Benutzer vorzusehen. Dies kann Situationen umfassen, in denen die Benutzer unterschiedliche Orientierungen (Drehorientierung) des jeweils angezeigten Bildausschnitts 116 auf der ihnen zugeordneten Wiedergabeeinheit nutzen. Sodann kann das jeweilige Eingabegerät 244, 252 bzw. dessen Koordinatensystems 248, 256 mit der jeweiligen Orientierung des Bildausschnitts in Übereinstimmung gebracht werden, um den Bildausschnitt intuitiv verschieben zu können. Dies kann die Bedienung für verschiedene Bediener deutlich vereinfachen. Gerade im medizinischen Umfeld ist es vorstellbar, dass verschiedene Personen an einer medizinischen Prozedur beteiligt sind. Demgemäß ist es vorstellbar, die Verantwortung für die Bedienung der Handhabungsvorrichtung 10 bzw. der robotischen Handhabungseinheit 34 im zeitlichen Ablauf einer medizinischen Prozedur zwischen den Beteiligten zu wechseln.

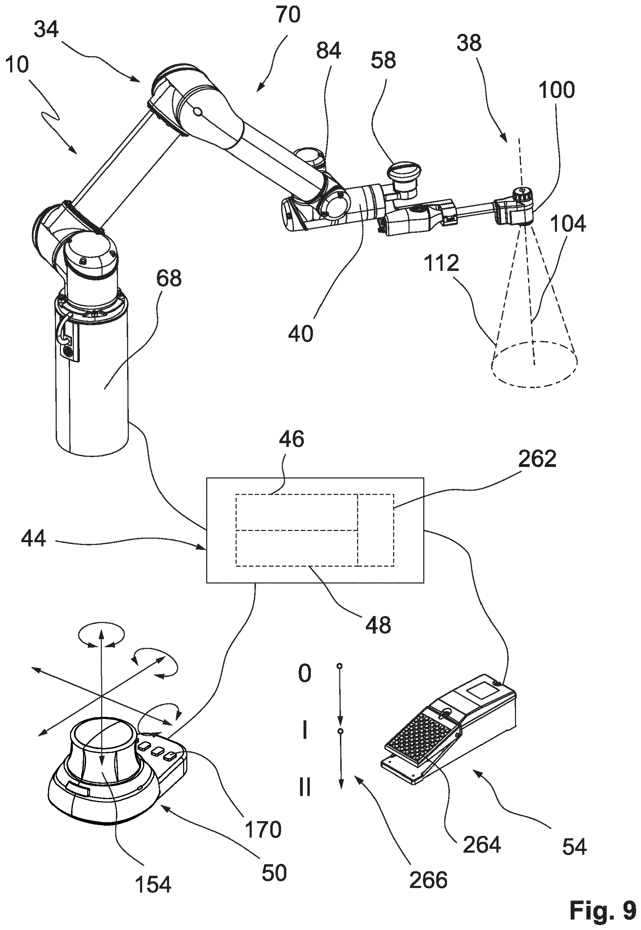

Fig. 9 veranschaulicht in Zusammenschau mit Fig. 1 einen Freigabeprozess zur Erhöhung der Sicherheit bei der Bedienung der Handhabungsvorrichtung 10, insbesondere der robotischen Handhabungseinheit 34. Zu diesem Zweck ist eine Sicherheitseinrichtung 262 vorgesehen, die auch als Freigabesteuerung bezeichnet werden kann. Die Sicherheitseinrichtung 262 kann als Bestandteil der Steuereinrichtung 44 ausgebildet sein, vergleiche die schematische Darstellung der Steuereinrichtung 44 mit der Handhabungssteuereinheit 46 und der Instrumentensteuereinheit 48 in Fig. 9.

Über das Eingabegerät 50 kann die robotische Handhabungseinheit 34 angesteuert werden, so dass Glieder der kinematischen Kette 70 bewegt werden, um das Beobachtungsinstrument 38 zu verfahren. Dies kann im Falle von Irrtümern oder Fehlbedienungen beträchtliche Auswirkungen haben. Demgemäß ist in beispielhaften Ausführungsformen ein zusätzlicher Freigabeprozess unter Nutzung eines Freigabeschalters vorgesehen. Beispielhaft handelt es sich bei dem Freigabeschalter um das Eingabegerät 54 bzw. um dessen Eingabeelement 264. Das Eingabeelement 264 ist beispielsweise als Fußschalter ausgebildet. Der Freigabeschalter hat eine Doppelfunktion, da er zum einen die Bewegung der robotische Handhabungseinheit 34 freigibt und zum anderen zum Hin- und Herschalten zwischen der Steuerung der Beobachtungseinheit 38 und der robotische Handhabungseinheit 34 nutzbar ist.

Es versteht sich, dass grundsätzlich auch das Eingabegerät 56 (vergleiche Fig. 1), welches an der Plattform 22 angeordnet ist, als Freigabeschalter nutzbar ist. Demgemäß kann die Sicherheitseinrichtung 262 derart gestaltet sein, dass eine Steuerung der Handhabungseinheit 34 nur dann möglich ist, wenn ein entsprechender Modus über das Eingabegerät 54 aktiviert ist.

Die Sicherheitseinrichtung 262 stellt sicher, dass nur bewusste Manipulationen der gegenwärtigen Position des Beobachtungsinstruments 38 möglich sind. Fehlbedienungen/unbewusste Steuerungen können vermieden werden.

In beispielhaften Ausführungsformen ist das Eingabegerät 54 mit der Sicherheitseinrichtung 262 fest verdrahtet. Mit anderen Worten kann es sich bei der Sicherheitseinrichtung 262 um eine diskrete Sicherheitseinrichtung handeln, welche nicht nur mittels Software bei der Steuereinrichtung 44 implementiert ist. Auf diese Weise ist die Sicherheitseinrichtung 262 unabhängig gestaltet. Manipulationen werden erschwert. Eine feste Ankopplung des Eingabegerätes 54 (fest verdrahtet bzw. "hard wired") erschwert Manipulationen desselben.

Das Eingabegerät 54 bzw. dessen Eingabeelement 264 weist/weisen zumindest zwei Schaltstellungen auf. Eine erste Schaltstellung (Stufe 0) entspricht beispielsweise einem unbetätigten Zustand. In diesem Zustand kann das Eingabegerät 50 nicht genutzt werden, um die Handhabungseinheit 34 zu steuern und zu bewegen. Das Eingabegerät 50 ist deaktiviert, zumindest im Hinblick auf die Steuerung der Handhabungseinheit 34. Eine zweite Schaltstellung (Stufe I) kann einen Zustand herbeiführen, in dem das Eingabegerät 50 aktiviert ist, so dass Befehle am Eingabegerät 50 von der Steuereinrichtung 44 verarbeitet werden, um die Handhabungseinheit 34 zu steuern und zu bewegen.

Zur weiteren Erhöhung der Sicherheit ist zumindest in beispielhaften Ausgestaltungen eine dritte Stufe (Stufe II) vorgesehen, welche auch als Panik-Modus/Panik-Stufe bezeichnet werden kann. Die zweite Stufe (Stufe I) ist bei dieser Ausgestaltung zwischen der ersten Stufe (Stufe 0) und der dritten Stufe (Stufe II) vorgesehen. Dies heißt mit anderen Worten, der Bediener muss eine bestimmte Mindestkraft aufbringen, um das Eingabeelement 264 des Freigabeschalters aus der ersten Stufe (Deaktivierungszustand) in die zweite Stufe (Aktivierungszustand) zu bringen. Diese Betätigungskraft darf jedoch eine definierte Maximalkraft nicht überschreiten. Beim Überschreiten der Maximalkraft wird nämlich das Eingabeelement 264 des Freigabeschalters aus der zweiten Stufe (Aktivierungszustand) in die dritte Stufe (Deaktivierungszustand oder Panik-Zustand) gebracht. Es muss also eine definierte Betätigungskraft aufgebracht werden, die in einem Bereich zwischen einer Minimalkraft und einer Maximalkraft liegt, um die robotische Handhabungseinheit 34 über das Eingabegerät 50 steuern zu können.

Diese Gestaltung erhöht die Sicherheit weiter. Nämlich dann, wenn der Freigabeschalter in Form des Eingabegerätes 54 unbeabsichtigt mit hoher Kraft betätigt wird, wird nicht zwangsläufig auch das Eingabegerät 50 für Bedienbefehle freigegeben. Stattdessen wird der Aktivierungszustand/Freigabezustand (zweite Stufe bzw. Stufe I) durchlaufen bzw. übersprungen und das Eingabegerät 54 in die dritte Stufe (Stufe II) versetzt.

Es versteht sich, dass der Freigabeschalter auch anderweitig gestaltet sein kann. So ist es vorstellbar, den Freigabeschalter bewusst nur mit einer ersten Stufe (Stufe 0) und einer zweiten Stufe (Stufe I) zu betreiben. Weitere Betätigungselemente können vorgesehen sein, etwa zusätzliche Bedienelemente, welche zusammen mit dem Freigabeschalter betätigt werden müssen. In der in Fig. 9 gezeigten Ausgestaltung ist der Freigabeschalter als Eingabegerät 54 in Form eines Fußschalters gestaltet. Es versteht sich, dass auch handbetätigte Schalter als Freigabeschalter nutzbar sind.

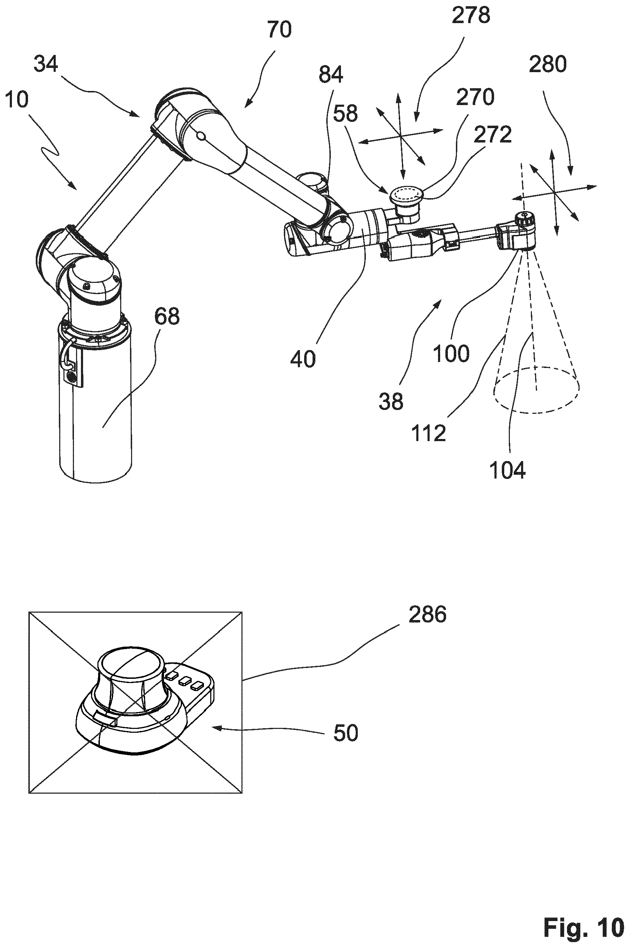

Fig. 10 veranschaulicht einen weiteren beispielhaften Betriebsmodus der Handhabungsvorrichtung 10, insbesondere der Handhabungseinheit 34. Der in Fig. 10 veranschaulichte Betriebsmodus kann auch als Direktsteuermodus bezeichnet werden. Im Direktsteuermodus erfolgt keine Steuerung der robotischen Handhabungseinheit 34 über das Eingabegerät 50, vergleiche den mit 286 bezeichneten durchgestrichenen Block in Fig. 10.

Stattdessen erfolgt im Direktsteuermodus die Steuerung der Handhabungseinheit 34 direkt durch eine Manipulation beziehungsweise einen Angriff an einem Element der kinematischen Kette 70 der Handhabungseinheit 34. Zu diesem Zweck ist in dem in Fig. 10 gezeigten Ausführungsbeispiel das Eingabegerät 58 vorgesehen, welches ein Eingabeelement 270 in Form eines Griffs oder Knopfs aufweist. Der Bediener kann das Eingabeelement 270 ergreifen und im Raum bewegen. Das Eingabegerät 58 mit dem Eingabeelement 270 ist beispielhaft am Instrumentenhalters 40 der robotischen Handhabungseinheit 34 angeordnet, also in unmittelbarer Nähe des aufgenommenen Beobachtungsinstruments 38. Das Eingabegerät 58 kann auch als Direktsteuereingabegerät bezeichnet werden.

Zumindest in einer beispielhaften Ausführungsform weist das Eingabegerät 58 beim Eingabeelement 270 einen Sensor 272 auf, der beispielsweise eine Annäherung oder ein Vorhandensein der Hand des Bedieners erfasst. Auf diese Weise kann die Steuerung der Handhabungseinheit 34 über das Eingabegerät 58 im Direktsteuermodus freigegeben werden.

Es ist vorstellbar, für die Freigabe des Direktsteuermodus unter Nutzung eines Sensors 272 beim Eingabeelement 270 des Eingabegerätes 58 auf eine zusätzliche Freigabe (vergleiche das als Freigabeschalter genutzte Eingabegerät 54 in Fig. 1 und Fig. 9) zu verzichten. Dies ist deshalb vorstellbar, weil der Bediener über das Eingabegerät 58 direkt oder nahezu direkt Einfluss auf die Position und Orientierung des am Instrumentenhalter 40 aufgenommenen Beobachtungsinstruments 38 nehmen kann. In einem solchen Fall ist die Bedienung eindeutig und unmittelbar möglich.

Im Direktsteuermodus werden Kraft- und Wegimpulse, die der Bediener auf das Eingabeelement 270 des Eingabegerät 58 aufbringt, von der Handhabungssteuereinheit 46 (vergleiche Fig. 1) erfasst und ausgewertet, wobei Antriebe der Elemente der robotischen Handhabungseinheit 34 derart angesteuert werden, dass die robotische Handhabungseinheit 34 mit dem Instrumentenhalters 40 und dem daran aufgenommenen Beobachtungsinstrument 38 der induzierten Bewegung am Eingabegerät 58 folgt. Es versteht sich, dass im Direktsteuermodus sowohl translatorische Bewegungen als auch rotatorische Bewegungen/Schwenkbewegungen des an der Handhabungseinheit 34 aufgenommenen Beobachtungsinstruments 38 bewirkt werden können.

Die Erfassung der Bedienimpulse kann etwa durch Überwachung der verschiedenen Achsantriebe der kinematische Kette 70 der Handhabungseinheit 34 erfolgen. Die Bedienimpulse sind in den Achsantrieben spürbar und können folglich erfasst werden. Alternativ können den Achsantrieben entsprechende Sensoren zugeordnet werden.

Alternativ oder zusätzlich ist es vorstellbar, das Eingabegerät 58 ähnlich dem Eingabegerät 50 mit eigenen Bewegungsfreiheitsgraden und entsprechenden Sensoren zu versehen, um Auslenkungen zu erfassen. Es auch vorstellbar, Kraftsensoren/Deformationssensoren beim Eingabegerät 58 bzw. bei dessen Eingabeelement 270 vorzusehen, um zu erfassen, wie der Bediener die Handhabungseinheit 34 mit dem Beobachtungsinstrument 38 bewegen möchte.

Die Steuereinrichtung 44, insbesondere die Handhabungssteuereinheit 46, steuert im Direktsteuermodus die Handhabungseinheit 34 derart, dass diese den Bewegungsimpulsen des Bedieners am Direktsteuer-Eingabegerät 58 folgt, und dass dann, wenn der Bediener nicht mehr auf das Eingabegerät 58 einwirkt, die aktuelle Position und/oder Orientierung beibehalten wird. Auf diese Weise kann der Bediener das Beobachtungsinstrument 38 quasi-manuell bewegen, wobei eine unmittelbare und direkte Rückkopplung erfolgt.

In Fig. 10 ist dem Eingabeelement 270 des Eingabegerätes 58 ein Eingabe-Koordinatensystem 278 zugeordnet. Ein hierzu ausgerichtetes Koordinatensystem 280 veranschaulicht die resultierende interpolierte Bewegung zum Verfahren des Beobachtungsinstruments 38 über Bedienimpulse am Eingabegerät 58. Das Beobachtungsinstrument 38 folgt den Bedienimpulsen am Eingabegerät 58 aufgrund der Nähe zwischen dem Eingabegerät und dem Beobachtungsinstrument 38 unmittelbar.

Die Handhabungssteuereinheit 46 kann derart betrieben werden, dass der Bediener im Direktsteuermodus einen bestimmten, aber nicht zu großen Widerstand (Bremsmoment) bei der Betätigung am Eingabegerät 58 verspürt. Dies erlaubt eine feinfühlige Bewegungs- und Positionsvorgabe im Direktsteuermodus.

Es ist ferner vorstellbar, im Direktsteuermodus durch die Steuereinrichtung 44, insbesondere durch deren Handhabungssteuereinheit 46, den durch den Bediener manuell über das Eingabegerät 58 gesteuerten Bewegungspfad der Handhabungseinrichtung 34 aufzuzeichnen und bedarfsweise "rückwärts" abzufahren. Auf diese Weise kann die Steuereinrichtung 44, insbesondere deren Handhabungssteuereinheit 46, eine Rückkehrfunktion bereitstellen. Das Gerät kann folglich die Startposition oder eine andere Position, welche gespeichert wurde, ausgehen von der gegenwärtigen Position anfahren. Dies ist grundsätzlich auch in anderen Steuermodi vorstellbar, nicht nur im Direktsteuermodus. Das Speichern und Aufrufen gewählter Funktionen kann beispielsweise über die Betätigungselemente 170 am Eingabegerät 50 oder über andere Betätigungselemente gesteuert werden.

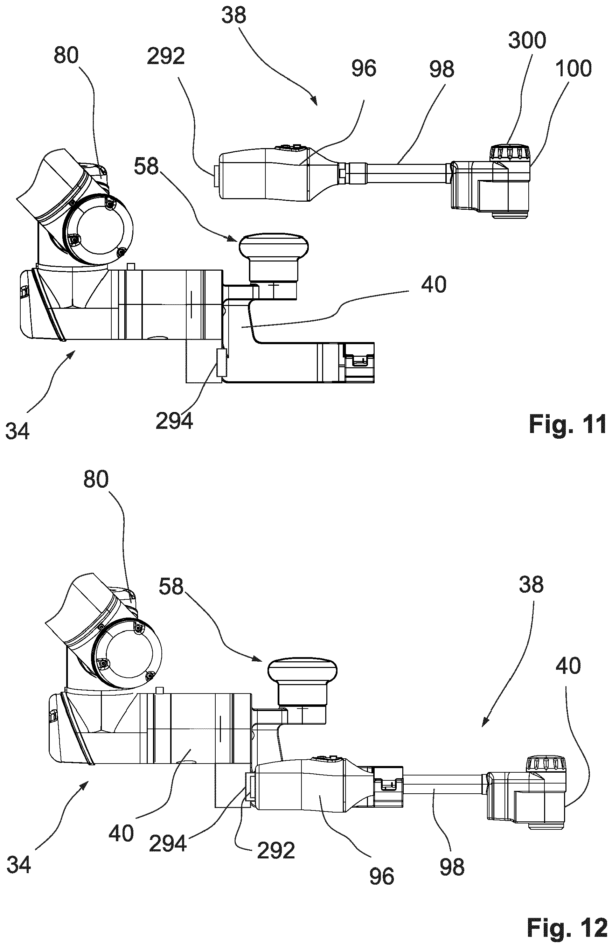

Die Figuren 11 und 12 veranschaulichen die Befestigung des Beobachtungsinstruments 38 am Instrumentenhalter 40 der Handhabungseinheit 34. In Fig. 11 ist das Beobachtungsinstrument 38 vom Instrumentenhalter 40 gelöst. In Fig. 12 ist das Beobachtungsinstrument 38 am Instrumentenhalter 40 befestigt. Neben der mechanischen Kopplung zwischen dem Beobachtungsinstrument 38 und dem Instrumentenhalters 40 ist ferner eine signaltechnische Kopplung über Schnittstellen 292, 294 vorgesehen. Eine Schnittstelle des Beobachtungsinstruments ist mit 292 bezeichnet. Eine Schnittstelle auf Seiten der Handhabungseinheit 34 ist mit 294 bezeichnet. Demgemäß kann gemeinsam mit der mechanischen Kopplung auch eine signaltechnische Kopplung erfolgen.

In Zusammenhang mit der Befestigung des Beobachtungsinstruments 38 kann folglich die Steuereinrichtung 44 der Handhabungsvorrichtung 10 über die Schnittstellen 292, 294 ermitteln, um welchen Instrumententyp es sich bei dem Beobachtungsinstrument 38 handelt. Eine solche Identifikation kann für eine instrumententyp-spezifische Grundeinstellung (Parametersatz) der Handhabungseinheit 34 genutzt werden. Dies kann etwa eine Berücksichtigung gegebener Abmessungen des Beobachtungsinstruments bei der Steuerung der robotischen Handhabungseinheit 34 betreffen. Ferner ist es vorstellbar, über die Schnittstellen 292, 294 Informationen betreffend einen Drehantrieb 300 für den Bildaufnehmer (in Fig. 11 und 12 nicht explizit dargestellt) am Beobachtungskopf 100 auszutauschen. Ferner ist es vorstellbar, Informationen betreffend eine aktuelle Orientierung/Drehlage des Bildaufnehmers zu erlangen.

Über die Schnittstellen 292, 294 können ferner Bildinformationen übertragen werden, also etwa Bildsignale des überwachten Bildausschnitts bzw. Aufnahmebereichs. Zusätzlich werden Informationen übertragen, die für den Betrieb der robotischen Handhabungseinheit 34 nutzbar sind.

Es ist vorstellbar, dass das Beobachtungsinstrument 38 eine Identifikationsinformation (ID) enthält, die über die Schnittstelle 292, 294 abgefragt werden kann. Demgemäß könnte sodann die Steuereinrichtung 44 auf Basis dieser Informationen einen Parametersatz betreffend das Beobachtungsinstrument 38 abfragen, etwa bei einer Datenbank. Ferner ist es vorstellbar, dass das Beobachtungsinstrument 38 selbst diese Informationen über die Schnittstelle 292, 294 bereitstellt.

Die in den Figuren 11 und 12 veranschaulichte Gestaltung betrifft den Informationsaustausch zwischen dem Beobachtungsinstrument 38 und einerseits der Handhabungssteuereinheit 46 und andererseits der Instrumentensteuereinheit 48. Entsprechende Signale/Informationen werden unter Zwischenschaltung der Handhabungseinheit 34 übertragen.

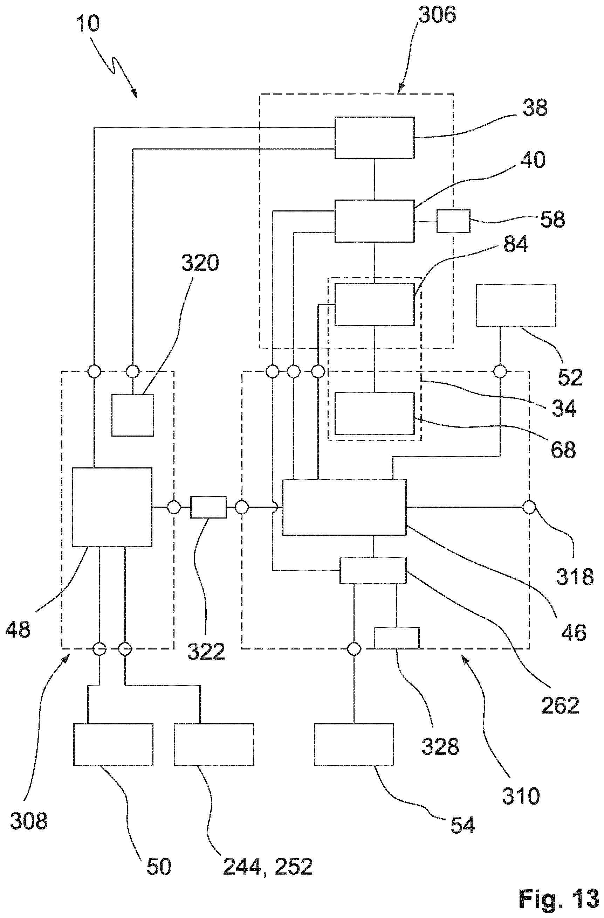

Fig. 13 zeigt anhand einer schematischen Darstellung ein Blockschaltbild zur Veranschaulichung einer beispielhaften funktionalen Ausgestaltung/Systemarchitektur einer Handhabungsvorrichtung 10. In struktureller Hinsicht kann die in Fig. 10 gezeigte Handhabungsvorrichtung 10 grundsätzlich der anhand der Figuren 1 bis 12 veranschaulichten Gestaltung der Handhabungsvorrichtung 10 entsprechen.

Die Handhabungsvorrichtung 10 weist einen Beobachtungsabschnitt 306, einen Instrumentensteuerungsabschnitt 308 und einen Handhabungsabschnitt 310 auf. Es handelt sich bei den Abschnitten 306, 308, 310 um funktional abgegrenzte Abschnitte. Es handelt sich nicht unbedingt um strukturell abgegrenzte Abschnitte. Der Beobachtungsabschnitt 306 umfasst das am Halter 40 aufgenommene Beobachtungsinstrument 38. Dem Beobachtungsabschnitt 306 ist ferner das Eingabegerät 58 für den Direktsteuermodus zugeordnet. Das Eingabegerät 58 weist beispielhaft einen Sensor zur Aktivierung des Direktsteuermodus auf.

Eine mechanische Verbindung des Beobachtungsabschnitts 306 mit dem Handhabungsabschnitt 310 erfolgt über die robotische Handhabungseinheit 34. Dort ist beispielsweise die Hand 84 vorgesehen, welche den Instrumentenhalter 40 trägt, vergleiche Fig. 2. Die Handhabungseinheit 34 umfasst ferner eine Basis bzw. ein Gestell 68, welches dem Handhabungsabschnitt 310 zugeordnet ist. Der Handhabungsabschnitt 310 umfasst ferner die Handhabungssteuereinheit 46 zur Steuerung der Handhabungseinheit 34. Der Handhabungseinheit sechsten 40 ist eine Schnittstelle 318 zur Energieversorgung vorgesehen. Die Schnittstelle 318 kann auch zum Informationsaustausch und/oder zur Medienversorgung dienen.

Darüber hinaus ist dem Handhabungsabschnitt 310 - in struktureller Hinsicht - auch das Eingabegerät 52 zugeordnet, welches beispielhaft als Touch-Monitor gestaltet ist. Das Eingabegerät 52 ist mit der Handhabungssteuereinheit 46 und folglich auch mit der (globalen) Steuereinrichtung 44 gekoppelt. Der Handhabungsabschnitt 310 umfasst ferner die Sicherheitseinrichtung 262, welche auch als Freigabesteuerung bezeichnet werden kann. Die Sicherheitseinrichtung 262 ist beispielsweise mit dem als Freigabeschalter gestalteten Eingabegerät 54 gekoppelt. Signaltechnisch ist die Sicherheitseinrichtung 262 beispielsweise mit der Handhabungssteuereinheit 46 gekoppelt, um Betriebsmodi der Handhabungssteuereinheit 46 und folglich der Handhabungseinheit 34 zu aktivieren oder zu sperren.

In dem Ausführungsbeispiel gemäß Fig. 13 ist die Sicherheitseinrichtung 262 ferner mit einem Feststell-Sensor 328 gekoppelt, der überwacht, ob die die Handhabungseinheit 34 tragende Plattform 22 bzw. der Wagen 24 gebremst und gesichert ist. Dies ist zumindest in beispielhaften Ausgestaltungen auch eine Bedingung für eine Freigabe der Bewegung der Handhabungseinheit 34.

Der Instrumentensteuerungsabschnitt 308 umfasst im Wesentlichen die Instrumentensteuereinheit 48, also die CCU/Konsole zur Überwachung und Steuerung des Beobachtungsinstruments 38. Demgemäß ist die Instrumentensteuereinheit 48 über zumindest eine Signalleitung mit dem Beobachtungsinstrument 38 gekoppelt. Ferner ist im Ausführungsbeispiel gemäß Fig. 13 eine Lichtquelle 320 vorgesehen, welche zu Beleuchtungszwecken mit dem Beobachtungsinstrument 38 gekoppelt ist.

Fig. 13 veranschaulicht ferner Leitungen zum Signalaustausch bzw. Informationsaustausch zwischen dem Beobachtungsabschnitt 306, dem Instrumentensteuerungsabschnitt 308 und dem Handhabungsabschnitt 310

Ferner ist zumindest ein Eingabegerät 50, 244, 252 über eine geeignete Schnittstelle mit der Instrumentensteuereinheit 48 gekoppelt. Es ist möglich, eine Mehrzahl von Eingabegeräten 50, 244, 252 vorzusehen, so dass verschiedene Bediener die Handhabungsvorrichtung 10 steuern können. Die Instrumentensteuereinheit 48 und das zumindest eine Eingabegerät 50, 244, 252 sind derart gestaltet, dass diese auch in einem handgeführten Modus des Beobachtungsinstruments 38 zur Steuerung verwendbar sind. Mit anderen Worten koordiniert die Instrumentensteuereinheit 48 die Bildaufnahme und gegebenenfalls die Bildwiedergabe. Ebenso ist das zumindest eine Eingabegerät 50, 244, 252 zur Steuerung von Bildaufnahmeparametern und Bildwiedergabeparametern verwendbar.

Es ist jedoch gemäß der in Fig. 13 veranschaulichten Ausgestaltung vorgesehen, das zumindest eine mit der Instrumentensteuereinheit 48 gekoppelte Eingabegerät 50, 244, 252 zusätzlich auch zur Steuerung der robotischen Handhabungseinheit 34 zu nutzen. Zu diesem Zweck ist die Instrumentensteuereinheit 48 mit der Handhabungssteuereinheit 46 verbunden, beispielsweise über eine Schnittstelle 322 zum Informationsaustausch. Die Schnittstelle 322 kann auch als Netzwerk-Schnittstelle oder Datenbus-Schnittstelle bezeichnet werden.

Diese Konfiguration führt dazu, dass in einem Modus, in dem das oder die Eingabegeräte 50, 244, 252 nutzbar ist/sind, um die robotische Handhabungseinheit 34 zur Bewegung des daran aufgenommenen Beobachtungsinstruments 38 zu steuern, die Instrumentensteuereinheit 48 die entsprechenden Steuersignale nicht selbst umfänglich verarbeitet, sondern diese über die Schnittstelle 322 an die Handhabungssteuereinheit 46 weiterleitet bzw. durchleitet. Ein solcher Modus wird beispielsweise über das als Freigabeschalter 54 gestaltete Eingabegerät unter Nutzung der Sicherheitseinrichtung 262 freigegeben.

Ein Vorteil dieser Gestaltung ist, dass die Instrumentensteuereinheit 48 weiterhin unabhängig und selbstständig zur Steuerung des Beobachtungsinstruments 38 verwendbar ist, etwa in einem handgehaltenen/handgeführten Modus. Dies gilt auch für mögliche Eingabegeräte 50, die direkt mit der Instrumenten Steuereinheit 48 gekoppelt sind. Dies ist auch ohne Handhabungseinheit 34 sowie deren Handhabungssteuereinheit 46 möglich.

Gleichwohl können die erweiterten Nutzungsumfänge durch die Bereitstellung der robotischen Handhabungseinheit 34 unter Nutzung ein und derselben Eingabegerätes 50 gesteuert werden. Es ist also nicht unbedingt notwendig, für die Steuerung der Handhabungseinheit 34 ein zusätzliches Eingabegerät 50 vorzusehen. Stattdessen kann das Eingabegerät 50 in verschiedenen Modi zur Instrumentensteuerung und zur Handhabungssteuerung genutzt werden.

Dies ist insbesondere dann vorstellbar, wenn das Eingabegerät 50 ein Mehrachs-Eingabegerät ist. Derartige Eingabegeräte (vergleiche etwa eine sogenannte 3D-Maus) eignen sich gut für beide Steuermodi. Es ergibt sich der Vorteil, dass ein und dieselbe Anordnung für einen handgehaltenen Betrieb des Beobachtungsinstruments 38 und einen durch die robotische Handhabungseinheit 34 unterstützten Betrieb nutzbar ist. Es ist nicht unbedingt notwendig, zweimal in das Beobachtungsinstrument 38 und die diesem zugeordnete Instrumentensteuereinheit 48 zu investieren. Gleichwohl wird eine einfache und sichere Bedienung der erweiterten Anordnung der Handhabungsvorrichtung 10 mit der robotischen Handhabungseinheit 34 gewährleistet.

Ein weiterer Aspekt der vorliegenden Offenbarung betrifft die Nutzung von aktuellen Betriebsparametern bzw. allgemeinen Parametern des Beobachtungsinstruments 38 durch die Steuereinrichtung 44, insbesondere durch deren Handhabungssteuereinheit 46, für die Steuerung der robotischen Handhabungseinheit 34. Insbesondere sind beispielhafte Ausgestaltungen vorstellbar, bei denen die Fahrgeschwindigkeit der robotischen Handhabungseinheit 34 von Betriebsparametern des Beobachtungsinstruments 38 abhängig gemacht wird. Dies kann beispielsweise einen aktuellen Objektabstand betreffen. Bei einem großen Objektabstand kann eine hohe Verfahrgeschwindigkeit und bei einem kleinen Objektabstand eine kleine Verfahrgeschwindigkeit zur Bewegung des Beobachtungsinstruments 38 gewählt werden.

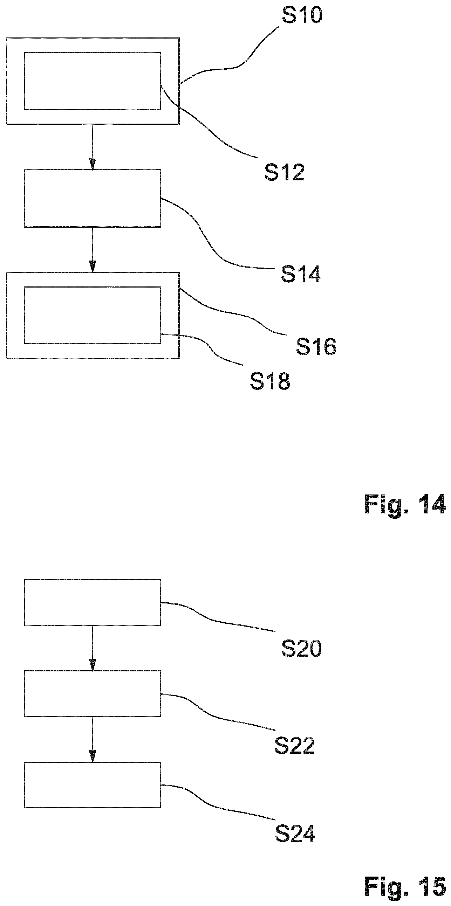

Mit Bezugnahme auf Fig. 14 wird anhand eines schematischen Blockdiagramms eine beispielhafte Ausführungsform eines Verfahrens zur Steuerung einer Handhabungsvorrichtung veranschaulicht. Bei der Handhabungsvorrichtung kann es sich um eine medizinische Handhabungsvorrichtung handeln. Es kann sich jedoch auch um eine nicht-medizinische Handhabungsvorrichtung handeln.

Das Verfahren umfasst einen Schritt S10, der die Bereitstellung eines Beobachtungsinstruments und dessen Anordnung an einem Instrumentenhalter an einer robotischen Handhabungseinheit umfasst. Der Schritt S10 umfasst ferner einen Teilschritt S12, der die Erfassung einer gegebenen Relativorientierung, insbesondere einer Ausrichtung (Drehorientierung) eines Bildaufnehmers umfasst. Ferner kann der Teilschritt S12 zumindest in beispielhaften Ausgestaltungen die Erfassung/Ermittlung einer allgemeinen Ausrichtung des Beobachtungsinstruments in Bezug auf den Instrumentenhalter umfassen. Dies kann beispielhaft vom erfassten Typ des Beobachtungsinstruments abgeleitet werden. Auf diese Weise kann ein Parametersatz ermittelt werden, auf dessen Basis Grundeinstellungen der Handhabungsvorrichtung vorgenommen werden können.

In einem Schritt S14 werden Steuerbefehle erfasst, die über ein Eingabegerät eingegeben werden, wobei die Steuerbefehle die Wahl eines wiederzugebenden Bildausschnitts betreffen. Mit anderen Worten können die Steuerbefehle ein Verschieben des anzuzeigenden Bildausschnitts zum Ziel haben. Andere Steuerbefehle sind denkbar. Insbesondere eignet sich ein Einhand-Eingabegerät zur Erfassung der Steuerbefehle. Beispielhaft ist das Eingabegerät als Mehrachs-Eingabegerät gestaltet. Das Eingabegerät ist sowohl zur Steuerung des Beobachtungsinstruments als auch zur Steuerung der robotischen Handhabungseinheit nutzbar.

In einem Schritt S16 wird die robotische Handhabungseinheit in Reaktion auf erfasste Bedienereingaben/Steuerbefehle gesteuert, um den erfassten Bildausschnitt zu ändern. Die Manipulation des Bildausschnitts kann beispielsweise ein Verschieben, Verdrehen und/oder Zoomen (Änderung des Abbildungsmaßstabs) umfassen. Dies umfasst beispielsweise einen Teilschritt S18, der die Überführung von Richtungsbefehlen am Eingabegerät in Bewegungsvorgaben umfasst, insbesondere in Bewegungsvorgaben für die robotische Handhabungseinheit. Dies erfolgt in Abhängigkeit von einer gegebenen Orientierung des Bildaufnehmers. Auf diese Weise kann eine Übereinstimmung zwischen einem Koordinatensystem/einer Orientierung des angezeigten Bildausschnitts und einem Koordinatensystems/einer Orientierung des Eingabegerätes herbeigeführt werden, unabhängig von der aktuellen Konfiguration und Orientierung der robotischen Handhabungseinheit. Die Bedienung kann sich vereinfachen. Der Bediener kann sich am angezeigten Bild orientieren und die Handhabungseinheit auf dieser Basis intuitiv steuern.

Fig. 15 veranschaulichten anhand eines Blockdiagramms eine weitere beispielhafte Ausgestaltung eines Verfahrens zur Steuerung einer Handhabungsvorrichtung. Das Verfahren umfasst einen Schritt S20, der die Erfassung einer Drehlage eines Bildaufnehmers eines Beobachtungsinstruments befasst. Bei dem Bildaufnehmer handelt es sich in einer beispielhaften Ausgestaltung um einen Stereo-Bildaufnehmer. Die Erfassung der Drehlage kann über einen Drehlagensensor und/oder mittelbar über die Steuerung eines Drehantriebs erfolgen.

In einem weiteren Schritt S22 erfolgt die Erfassung einer Bedienereingabe am Eingabegerät. Dies kann etwa eine Manipulation eines Mehrachs-Eingabeelements umfassen. Mit anderen Worten kann das Eingabeelement mehrere Freiheitsgrade für lineare Bewegungen und/oder Schwenkbewegungen aufweisen. Die Eingaben am Eingabegerät können beispielsweise darauf abzielen, den aktuell angezeigten Bildausschnitt zu verschieben, zu rotieren oder zu schwenken. Dies bedingt eine entsprechende Bewegung des durch die Handhabungseinheit gehaltenen Beobachtungsinstruments.

In einem weiteren Schritt S24 erfolgt zu diesem Zweck eine Koordinatentransformation, um die Bedienereingabe in Bewegungsvorgaben für die robotische Handhabungseinheit zu überführen. Dies erfolgt vorzugsweise unter Berücksichtigung der Drehlage des Bildaufnehmers. Somit ergibt sich eine Gestaltung, bei der sich ein Bediener am aktuell angezeigten Bildausschnitt orientieren kann, welcher die gegenwärtige Orientierung des Bildaufnehmers widerspiegelt. Der Bediener muss die aktuelle Ausrichtung der Handhabungseinheit und/oder des daran aufgenommenen Beobachtungsinstrument bei der Eingabe am Eingabegerät zur Manipulation des Bildausschnitts nicht gedanklich berücksichtigen. Dies erfolgt stattdessen automatisch im Hintergrund durch eine Koordinatentransformation.

Auf diese Weise kann auf Basis der Bedienereingabe, der erfassten Drehlage des Bildaufnehmers sowie der aktuellen Orientierung der Handhabungseinheit mit dem Beobachtungsinstrument in Reaktion auf die Bedienereingabe ein interpolierter Bewegungspfad für die Handhabungseinheit hergeleitet werden, auf dessen Basis die Handhabungseinheit gesteuert wird.

## Patentansprüche

1. Medizinische Handhabungsvorrichtung, die Folgendes aufweist:
- einen Instrumentenhalter (40) zur Aufnahme eines Beobachtungsinstruments (38) mit einem Bildaufnehmer (118) zur Erfassung eines Bildausschnitts (116),
- eine robotische Handhabungseinheit (34), die den Instrumentenhalter (40) trägt,
- eine Steuereinrichtung (44) mit einer Handhabungssteuereinheit (46) zur Steuerung der robotischen Handhabungseinheit (34) und einer Instrumentensteuereinheit (48) zur Steuerung des Beobachtungsinstruments (38), und
- ein mit der Steuereinrichtung (44) gekoppeltes Eingabegerät (50) zur Wahl eines wiederzugebenden Bildausschnitts (116),
wobei die Steuereinrichtung (44) dazu ausgebildet ist, die robotische Handhabungseinheit (34) in Reaktion auf Bedienereingaben am Eingabegerät (50) zu steuern, um den erfassten Bildausschnitt (116) zu ändern, und
wobei die Steuereinrichtung (44) dazu ausgebildet ist, Bedienbefehle am Eingabegerät (50) in Abhängigkeit von einer gegebenen Orientierung des Bildaufnehmers (118) in Bewegungsvorgaben zu überführen, insbesondere in Bewegungsvorgaben für die robotische Handhabungseinheit (34).

2. Handhabungsvorrichtung nach Anspruch 1, ferner aufweisend eine Wiedergabeeinheit (128) zur Anzeige des erfassten Bildausschnitts (116), wobei die Steuereinrichtung (44) die gegebene Orientierung des Bildaufnehmers (118) bei der Ansteuerung der Wiedergabeeinheit (128) zur Wiedergabe des Bildausschnitts (116) berücksichtigt.

3. Handhabungsvorrichtung nach Anspruch 1 oder 2, wobei die Steuereinrichtung (44) eine gegebene Ausrichtung des Bildaufnehmers (118) erfasst und in Abhängigkeit davon eine Zuordnung zwischen einem Eingabegerät-Koordinatensystem (156, 158) und einem Koordinatensystem (176, 178) der Handhabungseinheit (34) vornimmt, das die Orientierung des Bildaufnehmers (118) widerspiegelt.

4. Handhabungsvorrichtung nach einem der Ansprüche 1 bis 3, wobei das Beobachtungsinstrument (38) einen Orientierungssensor (142) zur Erfassung der Orientierung des Bildaufnehmers (118) aufweist, und/oder wobei das Beobachtungsinstrument (38) einen Stereo-Bildaufnehmer (118) mit zwei Bildsensoren (120, 122) aufweist.

5. Handhabungsvorrichtung nach einem der Ansprüche 1 bis 4, wobei der Bildaufnehmer (118) im Beobachtungsinstrument (38) drehbar ist, und/oder wobei die Steuereinrichtung (44) dazu ausgebildet ist, den durch den Bildaufnehmer (118) erfassten Bildausschnitt (116) digital zu rotieren.

6. Handhabungsvorrichtung nach einem der Ansprüche 1 bis 5, wobei die Steuereinrichtung (44) dazu ausgebildet ist, eine Zuordnung zwischen der Orientierung des Bildaufnehmers (118) und Bewegungsachsen (156, 158) für die Eingabe am Eingabegerät (50) derart vorzunehmen, dass Bewegungsrichtungen (186, 188) des durch die Wiedergabeeinheit (128) angezeigten Bildausschnitts (116) mit Richtungsvorgaben am Eingabegerät (50) in Übereinstimmung gebracht werden.

7. Handhabungsvorrichtung nach einem der Ansprüche 1 bis 6, wobei die Steuereinrichtung (44) dazu ausgebildet ist, die Bewegungsvorgaben in Steuerbefehle für Bewegungsachsen (74, 78, 82, 86, 88, 90) der robotischen Handhabungseinheit (34) zu überführen, und/oder wobei die robotische Handhabungseinheit (34) eine mehrgliedrige Kinematik (70) mit einer Mehrzahl von Koppelgliedern (72, 76, 80, 84) umfasst, welche von der Handhabungssteuereinheit (46) der Steuereinrichtung (44) gesteuert werden.

8. Handhabungsvorrichtung nach einem der Ansprüche 1 bis 7, wobei das Eingabegerät (50) als Mehrachs-Eingabegerät (50) gestaltet ist, vorzugsweise als Einhand-Mehrachs-Eingabegerät (50), um Bedienbewegungen in Form von Schubbewegungen oder Schwenkbewegungen in zumindest zwei Achsen (156, 158, 160, 162, 164, 166) erlaubt, um Bewegungssignale zur zweidimensionalen Bewegung des Bildausschnitts (116) in einer Ebene (180) zu erfassen, und wobei die Steuereinrichtung (44) vorzugsweise dazu ausgebildet ist, die zwei Bewegungsachsen (156, 158) des Eingabegerätes (50) mit der gegebenen Orientierung des Bildaufnehmers (118) auszurichten, so dass Bedienbewegungen eines Eingabeelements des Eingabegerätes (50) in richtungsgleichen Bewegungen (186, 188) des angezeigten Bildausschnitts (116) resultieren.

9. Handhabungsvorrichtung nach einem der Ansprüche 1 bis 8, wobei das Eingabegerät (50) als Einhand-Eingabegerät (50) gestaltet ist und Bedienbewegungen zumindest in Form einer Rotation (166) um eine Längsachse oder einer Translation (160) entlang der Längsachse erfasst, um Bewegungssignale zur Steuerung einer Zoom-Funktion sowie für eine Fokuseinstellung zu erfassen.

10. Handhabungsvorrichtung nach einem der Ansprüche 1 bis 9, wobei die Handhabungssteuereinheit (46) der Steuereinrichtung (44) dazu ausgestaltet ist, den angezeigten Bildausschnitt (116) in Reaktion auf Bedienbefehle am Eingabegerät (50) über eine Bewegung der robotischen Handhabungseinheit (34) zu bewegen, und wobei die Instrumentensteuereinheit (48) der Steuereinrichtung (44) dazu ausgestaltet ist, den angezeigten Bildausschnitt (116) in Reaktion auf Bedienbefehle am Eingabegerät (50) über eine digitale Verschiebung des angezeigten Bildausschnitts (116) in einem erfassten Aufnahmebereich (114) zu bewegen.

11. Handhabungsvorrichtung nach einem der Ansprüche 1 bis 10, wobei das Eingabegerät (50) in einem ersten Betriebsmodus zur Steuerung des Beobachtungsinstruments (38) und in einem zweiten Betriebsmodus zur Steuerung der robotischen Handhabungseinheit (34) betreibbar ist, und wobei die Handhabungsvorrichtung ferner einen Freigabeschalter (54) zur Aktivierung des zweiten Betriebsmodus aufweist, in dem die robotische Handhabungseinheit (34) in Reaktion auf Eingabebefehle am Eingabegerät (50) verfahrbar ist.

12. Handhabungsvorrichtung nach einem der Ansprüche 1 bis 11, wobei die Steuereinrichtung (44) dazu ausgestaltet ist, eine Initialisierungsprozedur durchzuführen, um Konfigurationsinformationen betreffend das aufgenommene Beobachtungsinstrument (38) zu erfassen, wobei die Initialisierung vorzugsweise eine Abfrage über die Instrumentensteuereinheit (48) aufweist, und wobei die Konfigurationsinformationen an die Handhabungssteuereinheit (46) übermittelt und bei der Steuerung der Handhabungseinheit (34) berücksichtigt werden.

13. Handhabungsvorrichtung nach einem der Ansprüche 1 bis 12, wobei die Steuereinrichtung (44) dazu ausgestaltet ist, den angezeigten Bildausschnitt (116) bedarfsweise zu spiegeln, wobei die Umsetzung von Bedienbefehlen am Eingabegerät (50) die Spiegelung berücksichtigt, und/oder wobei die Steuereinrichtung (44) dazu ausgestaltet ist, die Handhabungseinheit (34) derart anzusteuern, dass das Beobachtungsinstrument (38) durch interpolierte Bewegung der Handhabungseinheit (34) um eine virtuelle Kippachse verschwenkbar ist, die parallel zum Bildaufnehmer (118) angeordnet ist.

14. Handhabungsvorrichtung nach einem der Ansprüche 1 bis 13, wobei die Steuereinrichtung (44) dazu ausgestaltet ist, die robotische Handhabungseinheit (34) in einem Direktsteuermodus zu betreiben, um das Beobachtungsinstrument (38) im Raum zu bewegen und auszurichten, wobei Bedienbefehle an der robotischen Handhabungseinheit (34) durch Einwirkung auf ein dem Instrument benachbartes Element (40) der Handhabungseinheit (34) erzeugbar sind, und wobei die Handhabungssteuereinheit (46) dazu ausgestaltet ist, die robotische Handhabungseinheit (34) derart anzusteuern, dass das Beobachtungsinstrument (38) der induzierten Bewegung folgt, wobei die Bedienbefehle im Direktsteuermodus vorzugsweise über ein Bedienelement (270) abgegeben werden, welches über einen Sensor (272) ein Freigabesignal für den Direktsteuermodus erzeugt.

15. Verfahren zur Steuerung einer Handhabungsvorrichtung (10), die eine robotische Handhabungseinheit (34) mit einem Instrumentenhalter (40) und einem daran aufgenommenen Beobachtungsinstrument (38) mit einem Bildaufnehmer (118) zur Erfassung eines Bildausschnitts (116) umfasst, wobei das Verfahren die folgenden Schritte aufweist:
- Bereitstellung des Beobachtungsinstruments (38) am Instrumentenhalter (40),
- Erfassung von Steuerbefehlen zur Wahl eines wiederzugebenden Bildausschnitts (116) über ein Eingabegerät (50), das mit einer Steuereinrichtung (44) zur Steuerung des Beobachtungsinstruments (38) und zur Steuerung der robotischen Handhabungseinheit (34) gekoppelt ist, und
- Steuerung der robotischen Handhabungseinheit (34) in Reaktion auf Bedienereingaben am Eingabegerät (50), um den erfassten Bildausschnitt (116) zu ändern, umfassend:
- Überführung von Richtungsbefehlen am Eingabegerät (50) in Bewegungsvorgaben, insbesondere Bewegungsvorgaben für die robotische Handhabungseinheit (34), in Abhängigkeit von der gegebenen Orientierung des Bildaufnehmers (118).
